# EUROPEAN PATENT SPECIFICATION

(11) **EP 3 815 724 B1**
(45) Date of publication and mention of the grant of the patent: **07.06.2023**
(21) Application number: 20204880.7
(22) Date of filing: 30.10.2020
(51) Int. Cl.: A61M 1/16

(54) **BLOOD PROCESSING APPARATUS**
BLUTVERARBEITUNGSGERÄT
APPAREIL DE TRAITEMENT DU SANG

(30) Priority: 04.11.2019 KR 20190139273; 15.05.2020 US 202063025964 P; 13.08.2020 US 202063065480 P; 28.10.2020 US 202017082016
(43) Date of publication of application: 05.05.2021
(73) Proprietor: ExoRenal Inc., Baltimore, MA 21230 (US)
(72) Inventor: Lee, Jake Kyungsoo, Ellicott City, Maryland 21042 (US)
(74) Representative: Doherty, William

(56) References cited:
- KR-A- 20130 124 039
- US-A1- 2017 095 604
- US-A1- 2020 086 031

## Description

### TECHNICAL FIELD

The present invention relates to a blood processing apparatus, in which a plurality of fluid chambers are compressed and expanded simultaneously to allow blood and dialysis fluid to flow through a blood processing filter, thereby simplifying the apparatus, providing easy installation and operation, and reducing the cost for blood processing treatment.

### BACKGROUND

When there is a kidney dysfunction, water and waste products that have to be discharged out of body accumulate in blood and imbalance of electrolytes in the body occurs. Most commonly performed to improve such a kidney failure symptom, is hemodialysis which is to circulate blood out of body and rid the blood of the accumulated uremic toxin and excess water by a semi-permeable dialysis membrane. Hemodialysis is a method of seeking an electrolyte balance and ridding the body fluid of uremic toxin and excess water, taking advantages of diffusion applied due to the concentration difference and filtration applied due to the pressure difference between blood and dialysis fluid.

Hemodialysis is the example of the blood processing treatment in which blood of a patient is circulated extracorporeally to remove toxic substances from or supply beneficial ingredients to the blood. The blood processing treatment is frequently combined with a blood processing filter in which mass transfer between blood (i.e., a physiologic body fluid) and dialysis fluid (i.e., a purified sterile solution).

Most commonly used of blood processing filter is the type that is a cylinder-shaped container charged with a bundle of hollow fiber membranes and port-processed at both ends thereof by use of a synthetic resin like polyurethane. It is because the hollow fiber blood processing filter has excellent mass-transfer efficiency resulting from large effective surface area between blood and dialysis fluid compared to the small size as a whole.

Blood and dialysis fluid each decrease their hydraulic pressure while passing through a blood processing filter. Since blood and dialysis fluid flow in opposite directions inside the blood processing filter, a filtration occurs at the proximal part of the blood processing filter such that water in the blood moves toward dialysis fluid compartment because blood pressure is higher than dialysis fluid pressure, while a backfiltration occurs at the distal part such that water in the dialysis fluid moves toward blood domain for the same reason.

Conventional blood processing devices require a balancing unit connected to the multiple dialysis fluid tubes, two or more dialysis fluid pumps to transfer dialysis fluid, and a blood pump to transfer blood of a patient. Also, it is indispensable to disinfect the balancing unit, the dialysis fluid pumps, and the dialysis fluid flowing tubes on a regular basis, rendering the conventional blood processing unit complex in the structure and complicated to use. One example of a conventional dialysis machine known in the art is provided in US2017/095604A1. KR20130124039A discloses a hemodialysis apparatus for filtering impurities in blood by flowing blood and a dialysis liquid through a hemodialysis filter.

### SUMMARY

In order to solve the aforementioned problems, a blood processing apparatus is provided, in which multiple fluid chambers are compressed and expanded to transfer blood and dialysis fluid at the same time. The multiple chambers also ensure the flow rates upstream and downstream of the blood processing filter to be equally maintained. Thus, neither a separate blood pump nor a balancing chamber is required, and therefore, the entire system can be sufficiently miniaturized and light-weighted, and easy to be installed while reducing the cost for blood processing treatment. Thus, the blood processing apparatus will be an optimal alternative for the blood processing treatment in a place out of hospitals.

The blood processing apparatus comprises a fluid pumping unit having a plurality of fluid chambers each having an internal space, a chamber pressurizing member compressing or expanding the internal spaces of the multiple fluid chambers, a chamber pressurizing member driver driving the chamber pressurizing member, and a flow control unit.

The plurality of fluid chambers includes n fluid chambers, where n is 2 or more positive integer, the n fluid chambers are each connected with a first flow tube through which a fluid is provided to the chamber and a second flow tube through which a fluid of the chamber is discharged therefrom. The flow control unit controls a flow through the first and second flow tubes connected to the n chambers. When the n is an even number, n/2 chambers are compressed and another n/2 chambers are expanded simultaneously. When the n is an odd number, (n+1)/2 chambers are compressed and (n-1)/2 chambers are expanded simultaneously

The flow control unit controls the flow passages through the in-flow and out-flow tubes connected to the n fluid chambers. In one specific embodiment, the flow control unit may be formed of various valve structures, such as:
a one-way valve installed on each of the flow tubes connected to the n fluid chamber to allow a fluid to flow in one direction,
a solenoid valve installed on each of the flow tubes through which the flow control unit controls a flow to open or block a flow therethrough,
a pressurizing type valve having a flow-blocking member compressing a portion of the flow tubes through which the flow control unit controls a flow, a flow-blocking wall supporting the flow tubes compressed by the flow-blocking member, and a flow-blocking member driver providing a liner or curved movement force to the flow-blocking member, and
a rotating-type valve including a flow control housing having a cylinder-shaped internal space, a flow control rotor having a cylinder shape and disposed inside the internal space of the flow control housing, a plurality of flow control ports each penetrating the flow control housing between the internal space and an outer surface thereof, and a rotor driver driving the flow control rotor.

Here, the flow control unit according to an embodiment of the present invention may be configured to block a flow through approximately a half of the flow tubes through which the flow control unit controls a flow when the chambers are compressed or expanded.

Disclosed is the blood processing apparatus compressing and expanding multiple fluid chambers to transfer blood and dialysis fluid. The multiple chambers also ensure the flow rates upstream and downstream of the blood processing filter to be equally maintained. Thus, neither a separate blood pump nor a balancing chamber is required. Thus, the entire system can be sufficiently miniaturized and light-weighted, and easy to be installed while reducing the cost for blood processing treatment. This will make the blood processing apparatus suitable for an optimal alternative for the blood processing treatment in a place out of hospitals.

### BRIEF DESCRIPTION OF THE DRAWINGS

The accompanying drawings, together with the description, serve to explain principles of the present invention. In the drawings:
FIG. 1 is a view illustrating a schematic diagram of a blood processing apparatus according to an embodiment of the present invention;
FIGS. 2 and 3 are views illustrating circuit diagrams of a blood processing apparatus according to an embodiment of the present invention;
FIGS. 4 and 5 are views illustrating a fluid pumping unit of a blood processing apparatus according to an embodiment of the present invention;
FIG. 6 is a view illustrating a blood processing filter according to an embodiment of the present invention;
FIGS. 7 and 8 are views illustrating a flow control unit formed of a pressurizing type valve;
FIGS. 9 and 10 are views illustrating a flow control unit formed of a rotating type valve;
FIG. 11 is a view illustrating a circuit diagram of a blood processing apparatus in which a flow control unit is formed of a rotating type valve;
FIG. 12 is a view illustrating a flow control unit formed of a rotating type valve;
FIGS. 13 and 14 are views illustrating circuit diagrams of a blood processing apparatus having four (4) fluid chambers where two chambers are compressed, and another two chambers are expanded simultaneously;
FIG. 15 is a view illustrating a circuit diagram of a blood processing apparatus having three (3) fluid chambers;
FIG. 16 is a view illustrating a circuit diagram of a blood processing apparatus having four (4) fluid chambers where three chambers are compressed and one chamber is expanded simultaneously;
FIGS. 17 and 18 are views illustrating circuit diagrams of a blood processing apparatus having five (5) fluid chambers where three chambers are compressed and another two chambers are expanded simultaneously;
FIGS. 19 and 20 are views illustrating circuit diagrams of a blood processing apparatus having six (6) fluid chambers where three chambers are compressed and another three chambers are expanded simultaneously;
FIGS. 21 and 22 are views illustrating circuit diagrams of a blood processing apparatus having six (6) fluid chambers where three chambers are compressed and another three chambers are expanded simultaneously, where some chambers have a stoke volume that is different from another chambers;
FIGS. 23 and 24 are views illustrating circuit diagrams of a blood processing apparatus having six (6) fluid chambers where three chambers are compressed and another three chambers are expanded simultaneously;
FIGS. 25 and 26 are views illustrating circuit diagrams of a blood processing apparatus having six (6) fluid chambers where three chambers are compressed and another three chambers are expanded simultaneously, where a flow control unit is formed of a one-way valve;
FIGS. 27 and 28 are views illustrating circuit diagrams of a blood processing apparatus having eight (8) fluid chambers where four chambers are compressed and another four chambers are expanded simultaneously;
FIG. 29 is a view illustrating a circuit diagram of a blood processing apparatus having a blood pump;
FIG. 30 is a view illustrating a circuit diagram of a blood processing apparatus in which a plurality of fluid chambers is disposed in a vertical direction; and
FIG. 31 is a view illustrating a schematic diagram showing a method of controlling a flow according to an embodiment of the present invention.

### DETAILED DESCRIPTION OF PREFERRED EMBODIMENTS

Hereinafter, the blood processing apparatus according to the embodiments of the present invention will be described in detail with reference to the accompanying drawings.

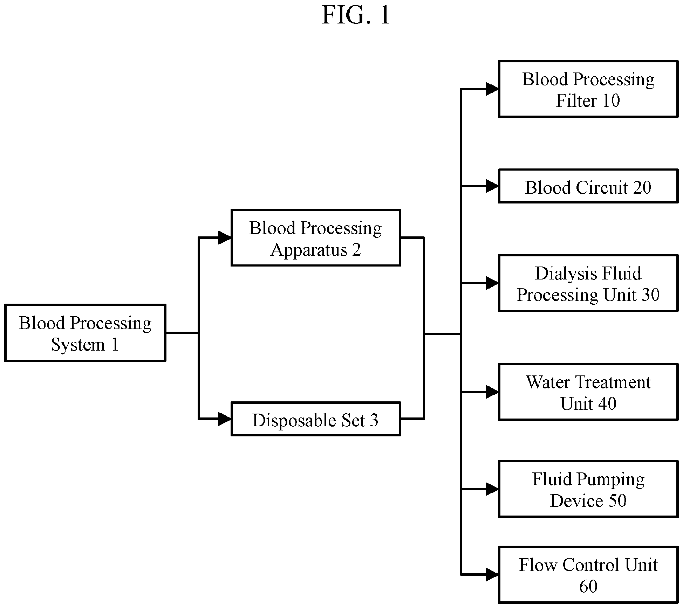

FIG. 1 is a schematic diagram of a blood processing apparatus 1 according to an embodiment of the present invention. The blood processing apparatus 1 according to the present invention is not limited to the device to preserve blood or the device to separate blood cells or plasma from whole blood, but it also includes any devices to provide treatments for patients, such as the device for hemodialysis to treat patients with end-stage renal disease (ESRD), the device for liver dialysis for patients with acute or acute-on-chronic liver failure, the extracorporeal life supporter (ECLS) device for replacing impaired functions of a lung and heart, or the purification device for treating patients with multiple organ failure requiring detoxification.

The blood processing apparatus 1 is configured to include a blood processing device and a disposable set. The blood processing device is a hardware unit having a housing in which various electric elements are mounted to perform the blood processing treatment. Software and programming to run the electric elements disposed in the blood processing device may be installed. The disposable set is a consumable element that is used for each treatment. Exemplary disposable unit includes tubes through which a fluid such as blood, dialysis fluid, or any biologic fluid flows, air drip chambers to remove air bubbles, and/or a blood processing filter.

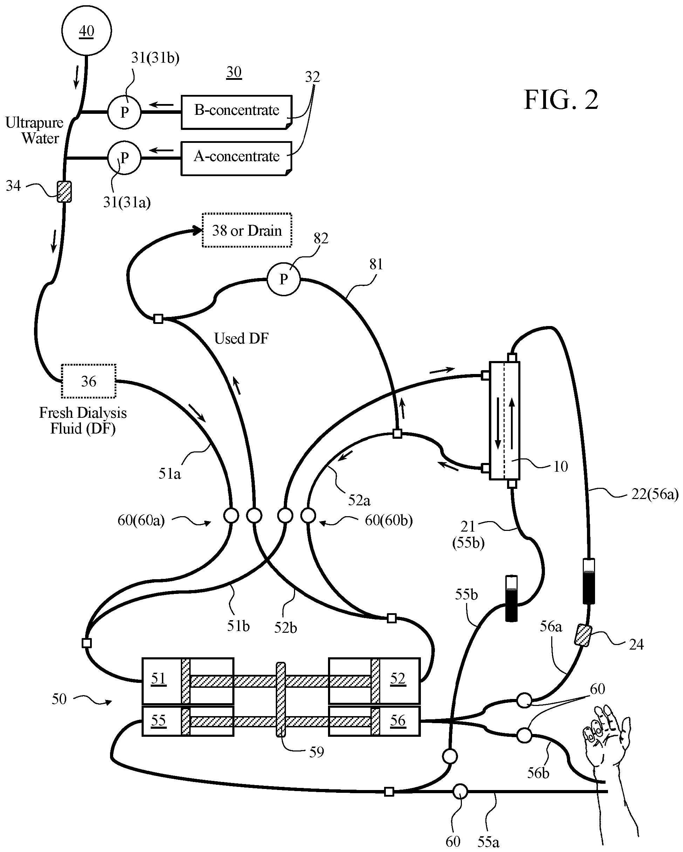

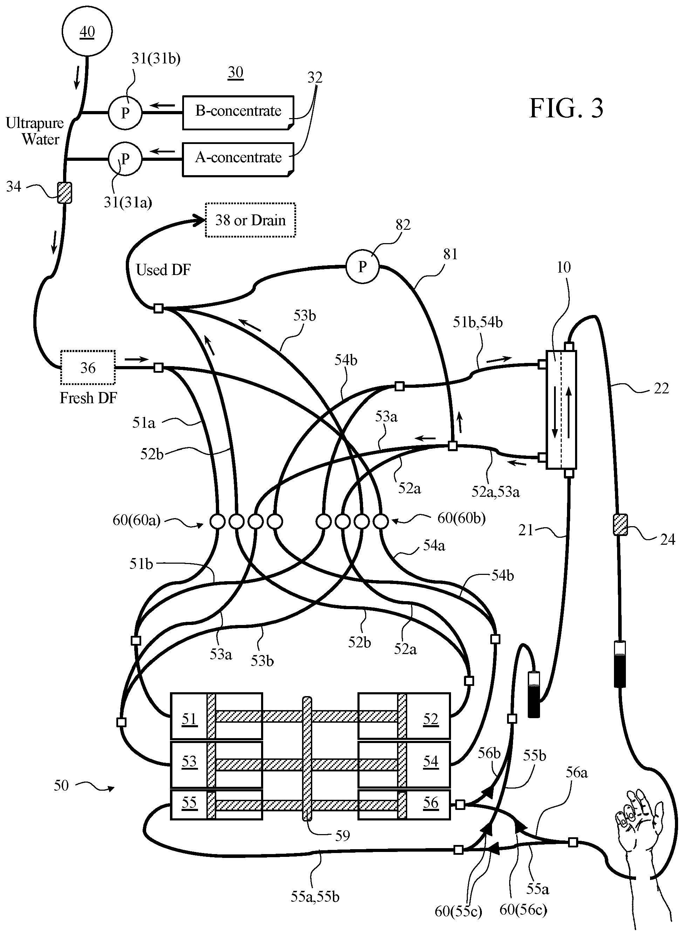

FIGS. 2 and 3 illustrate circuit diagrams of the blood processing apparatus 1. The blood processing apparatus 1 includes a fluid pumping unit 50 transferring blood and dialysis fluid, a dialysis fluid processing unit 30 to prepare fresh dialysis fluid by adjusting ion balance, a water treatment unit 40 generating ultrapure water, and a flow control unit 60 controlling flow passages through the fluid flowing tubes. Various safety and monitoring sensors 24 and 34 may also be provided to monitor the blood processing treatment. The blood processing apparatus 1 also includes the blood processing filter 10 in which blood is processed. For example, mass transfer occurs between blood and dialysis fluid in the blood processing filter 10.

The fluid pumping unit 50 includes a plurality of fluid chambers each having an internal space, a chamber pressurizing member 59 compressing or expanding the internal spaces of the fluid chambers, and a pressurizing member driver (not shown) operating the chamber pressurizing member 59. The plurality of fluid chambers include n fluid chambers, where n is a positive integer that is equal to and greater than 2. Preferably, the fluid pumping unit 50 may comprise three (3) to eight (8) fluid chambers. For example, FIGS. 2 and 3 illustrate exemplary circuit diagrams of the blood processing apparatus 1 having four and six fluid chambers (i.e., first to sixth fluid chambers 51 to 56), respectively.

Here, although the term 'dialysis fluid' is used to distinguish it from blood, the dialysis fluid is not limited to the fluid that is used for hemodialysis, continuous renal replacement therapy (CRRT), or peritoneal dialysis. The dialysis fluid is interpreted to include any fluids that can be used for any types of blood processing treatments including but not limited to plasma, serum, distilled water, isotonic saline solution, lactose solution, and others.

Each of the n fluid chambers is connected with an in-flow tube through which a fluid flows into the chamber and an out-flow tube through which a fluid contained in the chamber is discharged. For example, the first chamber 51 is connected with a first chamber in-flow tube 51a and a first chamber out-flow tube 51b. A fluid flows into the first chamber 51 through the first chamber in-flow tube 51a and a fluid may be discharged from the chamber through the first chamber out-flow tube 51b. In the same manner, the second chamber 52 is connected with a second chamber in-flow tube 52a and a second chamber out-flow tube 52b, which can be applied similarly to other chambers.

The in-flow and out-flow tubes are merely expressions to describe the tubes connected to the chamber and they shouldn't be interpreted that a fluid must flow into the chamber through the in-flow tube or leave the chamber through the out-flow tube. For example, a fluid flows into the chamber through the out-flow tube, or a fluid may be provided to or discharged from the chamber through both the in-flow and out-flow tubes. In addition, as shown in FIGS. 2 and 3, each chamber is connected with the in-flow and out-flow tubes, but the in-flow and out-flow tubes may overlap in a portion such that a single tube is connected to the chamber.

The n chambers are compressed or expanded simultaneously. That is, all of the n chambers may be compressed simultaneously, or expanded simultaneously. Alternatively, a portion of the n chambers may be compressed while the other portion of the n chambers are expanded. For example, when the fluid pumping unit 50 includes six chambers 51 to 56, all of the six chambers may be compressed at once (or expanded at once). Otherwise, three chambers are expanded simultaneously while the other three chambers are expanded. Alternatively, four chambers may be compressed while two chambers are expanded, and vice versa.

In FIGS. 2 to 3, the chambers are configured to have a cylinder-shaped internal space and the chamber pressurizing member 59 has a shape of a piston reciprocally disposed inside the cylinder-shaped chambers to compress or expand the chambers. However, the chamber and the chamber pressurizing member are not limited to the drawings. A container having an internal space to accommodate a fluid and any means that pressurizes or expands the internal space of the container to thereby allow a fluid to flow through the container can be used as the chamber and the chamber pressurizing member of the present invention. For example, a fluid sac, a fluid bag, or a fluid tube may be used for the chamber and any element pressurizing or expanding the fluid sac, the fluid bag or the fluid tube can be used as the chamber pressurizing member 59.

The chamber may be made of a substantially inflexible material having a predetermined shape, such as the cylinder shape, and the chamber pressurizing member 59 may have a portion that is made of a substantially flexible material such as rubber, polymer, silicone, and the like. Otherwise, the chamber can be made of a flexible material and the chamber pressurizing member 59 may have a portion that is inflexible to compress the flexible chambers.

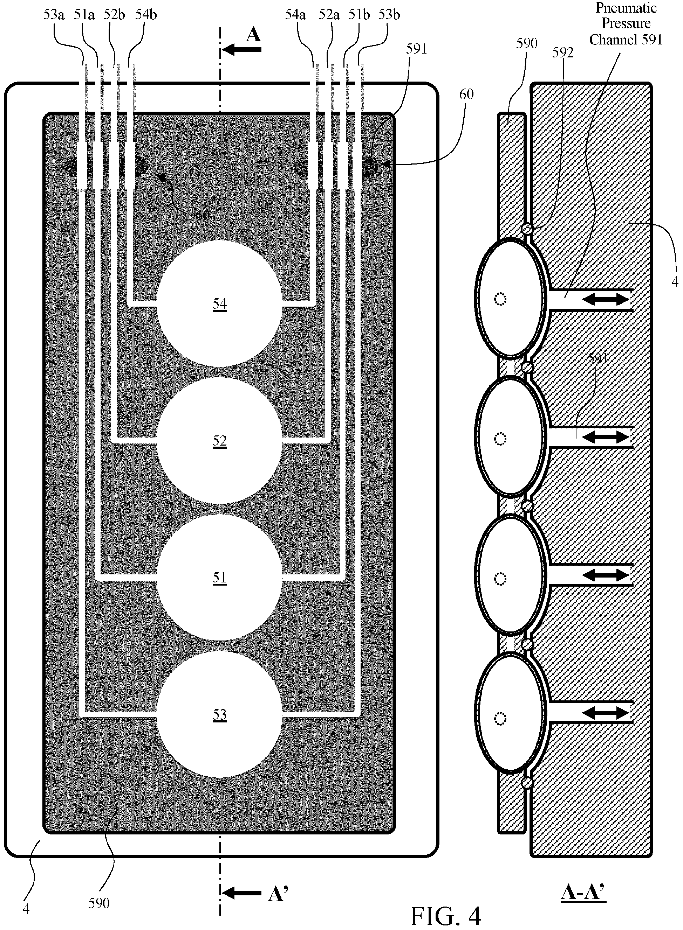

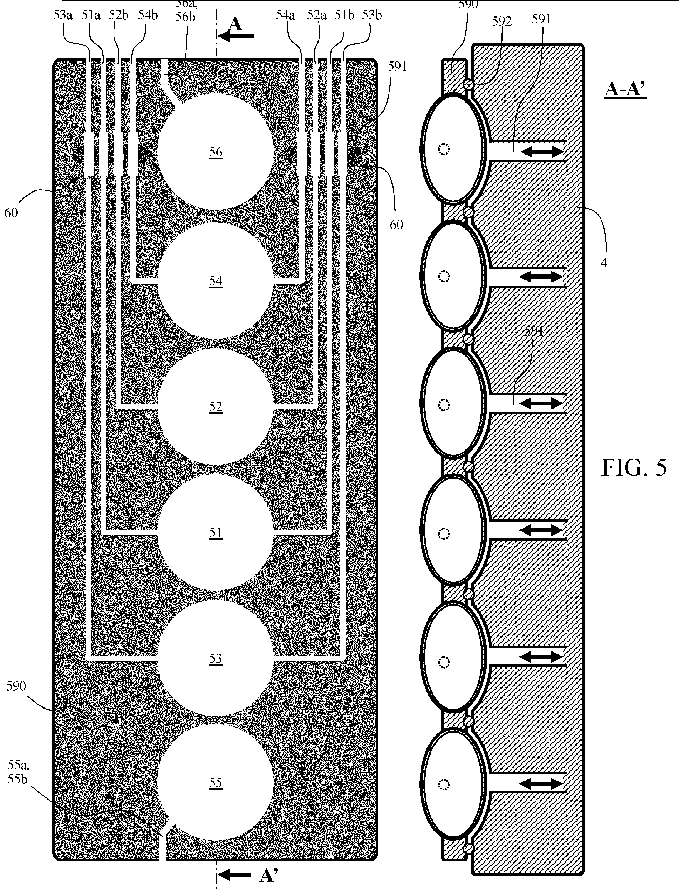

For example, FIGS. 4 and 5 illustrate the fluid pumping unit 50, in which the fluid chamber has a form of a fluid sac (or a fluid bag) made of a flexible material and having an internal space. The sacs may be installed inside a frame 590 as the frame 590 provides an installation space. The chamber pressurizing member 59 has a structure to pressurize or depressurize the fluid sacs. For example, the fluid sacs may be compressed or expanded by an operation of a pneumatic driver, such as a pneumatic pump, gas pump, vacuum pump, and others. The pneumatic driver placed in the housing 4 of the blood processing apparatus 1 compresses or decompresses the pneumatic channel 591 (which is connected to the outer space surrounding the fluid sacs), resulting in the compression or decompression of the fluid sacs. For example, the pneumatic channel 591 may serve as the chamber pressurizing member 59. A gasket 592 may be provided to prevent a pneumatic leakage around the fluid sacs. The gasket 592 can be made of a flexible material or an inflexible material such as plastic, metal, polymer, and others.

Since the chambers are expanded and compressed simultaneously, a single chamber pressurizing member 59 may be used for compressing and expanding the chambers. In this regard, a single chamber pressurizing member driver may be used to operate the chamber pressurizing member 59. The chamber pressurizing member driver includes various structures which allow the chamber pressurizing member 59 to reciprocate along a straight line or a curved line so as to compress or expand the internal spaces of the chambers. An exemplary chamber pressurizing member driver may include a cam pushing the chamber pressurizing member 59 in a rectilinear direction and a motor rotating the cam. Alternatively, the chamber pressurizing member driver may have a structure including a motor, a circular gear rotating by the motor, a linear gear moving along a straight line due to the rotation of the circular gear. Due to the rotation of the cam or circular gear, the chamber pressurizing member 59 moves along a rectilinear direction, and when the motor rotates further or rotates in an opposite direction, the chamber pressurizing member 59 may move to an opposite direction.

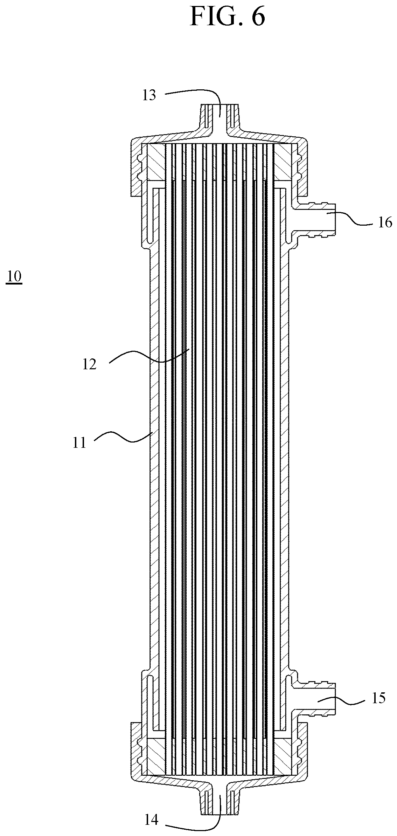

The blood processing filter 10 includes various filter apparatuses to process blood of a patient. As shown in FIG. 6, the blood processing filter 10 may have a form in which a blood processing membrane 12 is accommodated in the filter housing 11. The internal space of the filter housing 11 can be divided into multiple flow regions by the membrane 12, through which a separate fluid flows. In an embodiment, the blood processing filter 10 is divided into a blood flow region and a dialysis fluid flow region by the blood processing membrane 12.

The filter housing 11 is provided with a first blood port 13 and a second blood port 14 disposed at an opposite side thereof. Blood may enter the blood processing filter 10 through the first blood port 13 and leave therefrom through the second blood port 14. Blood tubes 21 and 22 may be connected to the blood ports 13 and 14, respectively, to allow blood to flow through blood processing filter 10. Also, a first dialysis fluid port 15 and a second dialysis fluid port 16 may be provided on the filter housing 11 to allow the dialysis fluid to flow through the blood processing filter 10. Specifically, dialysis fluid may be provided to the blood processing filter 10 through the first dialysis fluid port 15 and is discharged therefrom through the second dialysis fluid port 16.

Blood passes through the blood flow region inside the blood processing filter 10 and dialysis fluid passes through the dialysis fluid flow region. Blood and dialysis fluid may be desirably configured to flow in the opposite directions to each other. The blood processing filter 10 is not limited to the structure shown in the drawing, and may be modified into other forms including a hemodialyzer, an adsorption filter column, or a hemodiafilter.

Fresh dialysis fluid is produced through the dialysis fluid processing unit 30. Acid and bicarbonate ion solutions (or acid and bicarbonate powder) can be mixed with ultrapure water that is prepared through the water treatment unit 40. Through this process, ion concentrations such as bicarbonate, sodium, etc., and pH of the dialysis fluid can be adjusted.

The dialysis fluid processing unit 30 may be provided with dialysis fluid processing pumps 31 to transfer the acid and/or bicarbonate solution 32. The dialysis fluid processing pumps 31 may also include a first dialysis fluid processing pump 31a and a second dialysis fluid processing pump 31b to transfer first and second ion solutions 32. The dialysis fluid processing pump 31 needs to deliver the precise amount of solutions, and therefore a precise metering pump may be used for the dialysis fluid processing pump 31. Exemplary dialysis fluid processing pump 31 includes a rotary piston pump, a metering peristaltic pump, a precise piston pump, and the like.

A fresh dialysis fluid container 36 and/or a used dialysis fluid container 38 may be used to store fresh dialysis fluid and/or to collect used dialysis fluid, respectively. However, fresh dialysis fluid can be supplied to the blood processing filter 10 without being stored in the fresh dialysis fluid container 36 and the used dialysis fluid may be discarded without being collected in the used dialysis fluid container 38.

The dialysis fluid is not limited to be produced through the dialysis fluid processing unit 30. The dialysis fluid may be provided by using a pre-made dialysis fluid bag. In addition, the blood processing apparatus 1 may further be provided with a means 34 of measuring the purity of the fresh dialysis fluid, such as a conductivity sensor.

The water treatment unit 40 generates ultrapure water and includes multiple filtration stages, such as a pre-processing filter, a carbon filter, a reverse osmosis filter, ion-exchange resin beds, and/or an endotoxin retention filter. The water treatment unit 40 can be modified into a different configuration to prepare ultrapure water satisfying the requirement of the blood processing treatment.

The flow control unit 60 controls a flow (or a flow passage) through the in-flow and out-flow tubes. Thus, various valve structures that can open or close the flow through the tubes may be used for the flow control unit 60. For example, the flow control unit 60 may have a structure of a one-way valve, a solenoid valve, an on-off valve, a pressurizing type valve, a rotating-type valve, or a pneumatic valve. The flow control unit 60 may be formed of a combination of these valve types.

One-way valves installed on each of the flow tubes through which the flow control unit 60 ensure a fluid to flow in one direction. Solenoid valves and on-off valves may be installed on each of the flow tubes to open or block a flow therethrough.

The pneumatic valve or a pneumatic valve assembly may include a pneumatic driver and a pneumatic channel. The pneumatic driver pressurizes or depressurizes a pneumatic channel, thereby compressing or decompressing, i.e., blocking or opening, the flow tubes through which the flow control unit 60 controls a flow. Exemplary pneumatic flow control unit 60 is illustrated in FIGS. 4 and 5. Various types of pneumatic drivers can be used to pressurize or depressurize the pneumatic channel, as mentioned above.

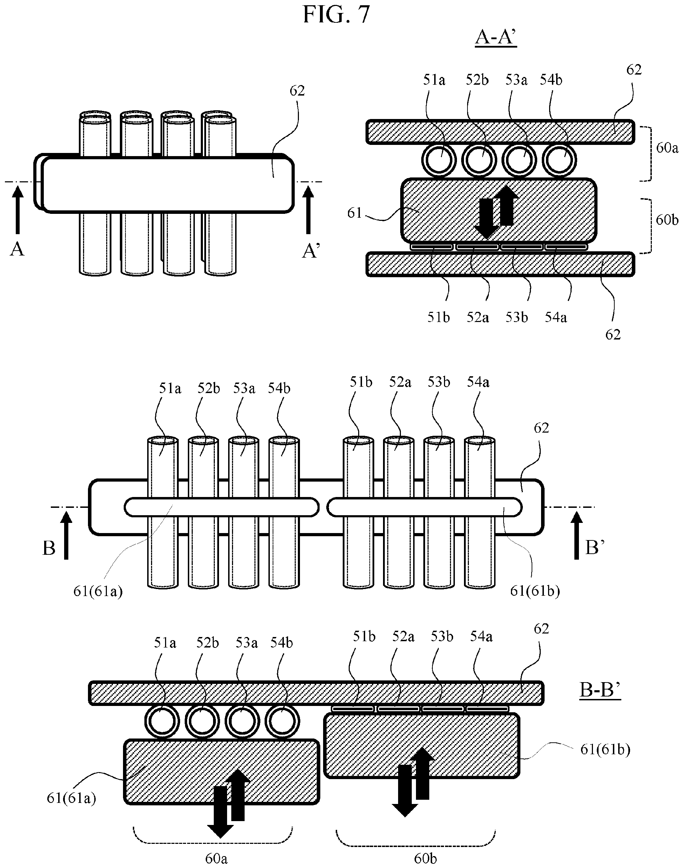

The pressurizing type valve is illustrated in FIGS. 7 to 9. The pressurizing type valve includes a flow blocking member 61 reciprocating in a straight line or in a curved line to compress a portion of the tubes through which the flow control unit 60 controls a flow, a flow blocking wall 62 supporting the tubes compressed by the flow-blocking member 61, and a flow-blocking member driver providing a straight or curved force to the flow-blocking member 61.

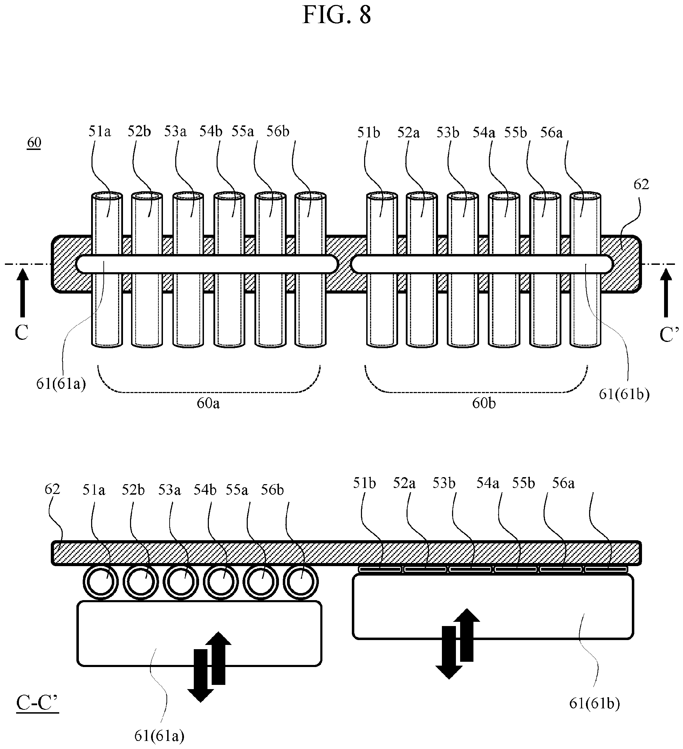

FIGS. 7 and 8 are views illustrate the exemplary flow control unit 60 regulating the flow through eight (8) flow tubes 51a, 51b, 52a, 52b, 53a, 53b, 54a and 54b, which are connected to the first to fourth chambers 51 to 54. The flow control unit 60 may block the flow through the tubes 51a, 52b, 53a, 54b and the tubes 51b, 52a, 53b, 54a in an alternate manner. When the flow-blocking member 61 moves to the tubes 51a, 52b, 53a and 54b, an end of the flow-blocking member 61 compresses the tubes 51a, 52b, 53a and 54b supported by the flow-blocking wall 62 and blocks the flow therethrough. At this time, the flow passages through the tubes 51b, 52a, 53b and 54a are opened. Similarly, the flow blocking member 61 moves to the tubes 51b, 52a, 53b and 54a, and another end of the flow-blocking member 61 compresses the tubes supported by the flow-blocking wall 62 and blocks the flow therethrough.

Although the flow-blocking member 61 is described to have an end and another end, the flow control unit 60 is not limited to the structure. For example, as shown in FIG. 7, the flow control unit 60 may be able to control the flow through the tubes 51a, 51b, 52a, 52b, 53a, 53b, 54a, and 54b using two or more flow-blocking members 61a and 61b which are separated from each other. In this case, two or more flow-blocking member drivers may be used to operate each of the flow-blocking members 61a and 61b.

Alternatively, when the tubes are made of flexible materials, such as rubber, silicone, polyurethane, polyacetate, other polymers, etc., it may be possible to bend the flow tubes by a predetermined angle to thereby block the flow passage through the flow tubes. The flow-blocking member 61 may not only compress the tubes to close the flow inside, but also bend the tubes to block the flow.

The flow-blocking member driver includes various structures that can apply a reciprocating movement force (that is, for a rectilinear or curvilinear movement) to the flow-blocking member 61. The same description made for the chamber pressurizing member driver above can be applied to the flow-blocking member driver. For example, an exemplary flow-blocking member driver may include a cam for pushing the flow-blocking member 61 toward the flow-blocking wall 62 supporting the tubes and a motor rotating the cam. When the flow-blocking member 61 compresses the tubes due to the rotation of the cam, the flow therethrough may be blocked. When an external force by the cam is removed, the flow-blocking member 61 may detach from the tube, and the tube may be restored to the original state by its own elastic force, expanding the inside of the tube. Or, an eccentric cam connected to a motor may rotate and compress one side of the tube and block the flow therethrough. The cam further rotates such that an external force applied by the cam may be removed and the tube is restored to its original status, expanding the inside of the tube.

The flow control unit 60 can be modified to control the flow through the in-flow and out-flow tubes connected to the first to sixth chambers 51 to 56, e.g., the tubes 51a, 51b, 52a, 52b, 53a, 53b, 54a, 54b, 55a, 55b, 56a, and 56b, as illustrated in FIG. 8. The flow control unit 60 may block the flow through the tubes 51a, 52b, 53a, 54b, 55a, 56b and the tubes 51b, 52a, 53b, 54a, 55b, 56a in an alternate manner. In order to hold the tubes that are pressurized by the flow blocking member 61, the flow control unit 60 may further include a tube holder 63 (not shown in the drawing).

Here, when the chambers are compressed or expanded, the flow control unit 60 may block the flow through approximately a half, or at least a half, of the flow tubes through which the flow control unit 60 controls a flow.

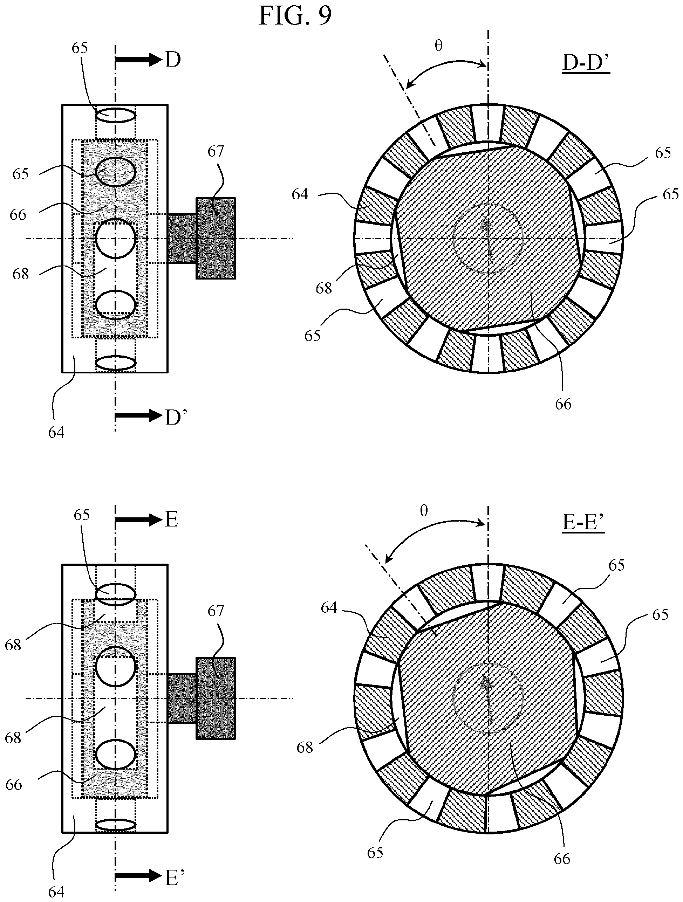

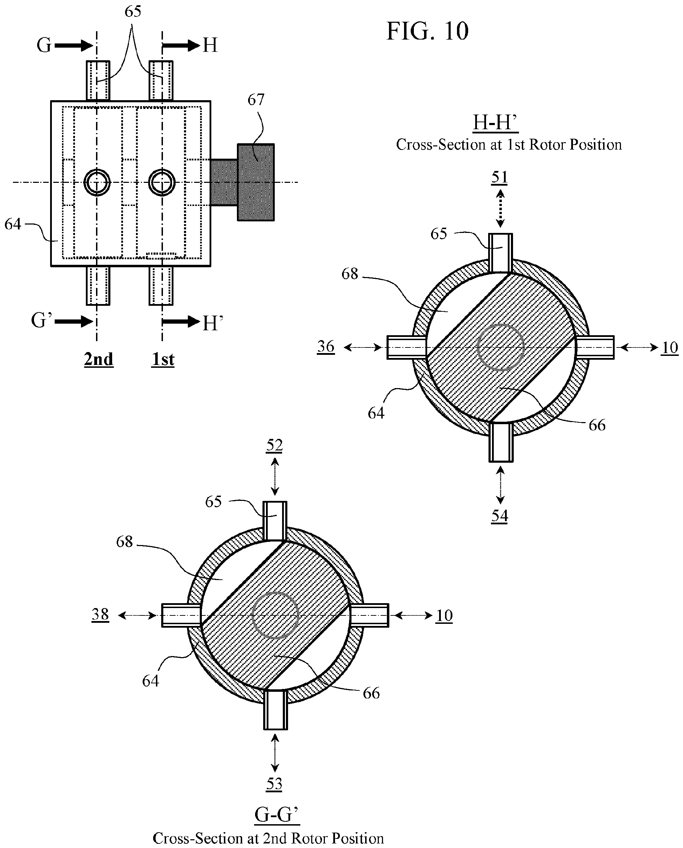

The flow control unit 60 is not limited to the structures described above, and may be modified into a structure of the rotating-type valve. As illustrated in FIGS. 9 and 10, the rotating-type valve includes a flow control housing 64 having an internal space, a flow control rotor 66 which is disposed inside the flow control housing 64, a plurality of flow control ports 65 disposed on the flow control housing 64 and penetrating the flow control housing 64, and a rotor driver 67 operating the flow control rotor 66.

The flow control rotor 66 and the internal space of the flow control housing 64 may have a cylindrical shape in order to allow the flow control rotor 66 to rotate inside the flow control housing 64. However, the flow control rotor 66 may be modified to move along a rectilinear direction. Further, the flow control rotor 66 may be able to rotate while moving along a rectilinear direction. Due to the rotation or linear movement of the flow control rotor 66, a flow passage can be connected between at least two flow control ports 65.

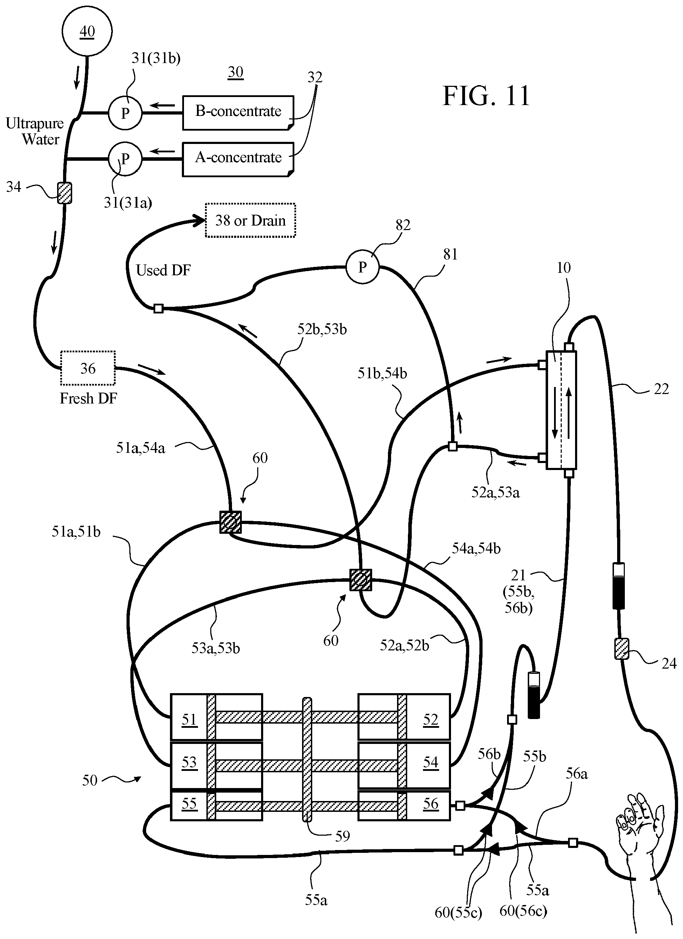

The flow control rotor 66 may also be formed with a recessed portion 68 to make it easier for a fluid to flow through two adjacent flow control ports 65. The recessed portion 68 may have a cross-sectional shape of a crescent moon, a rectangular, a square, a quadrilateral, or a triangular shapes. A circuit diagram of the blood processing apparatus 1 in which the flow control unit 60 is formed of the rotating-type valves is illustrated in FIG. 11.

The flow control ports 65 formed in the flow control housing 64 may be spaced apart along a circumferential direction of the internal space of the flow control housing 64 which has a cylinder shape. In addition, some of the flow control ports 65 may be placed within substantially the same cross-sectional plane which is perpendicular to an axial direction of the internal space of the flow control housing 64. For example, the flow control ports 65 of FIG. 9 are placed within substantially the same cross-sectional plane of D-D' or E-E'. Further, the flow control ports 65 may be placed in two or more separate cross-sectional planes of G-G' and H-H' as shown in FIG. 11. The flow control ports 65 can be placed at substantially the similar elevation along an axial direction of the flow control rotor 66.

The flow control rotor 66 rotates unidirectionally or bidirectionally to control the opening and blocking of the flow passage through the flow control ports 65. However, the flow control rotor 66 can move along a rectilinear direction or rotate while moving along a rectilinear direction. The time for opening or blocking the flow passage can be controlled by regulating the movement speed of the flow control rotor 66.

The flow control rotor 66 needs to be tightly attached to the inner surface of the flow control housing 64 to inhibit a fluid from leaking through the contact surface of the flow control rotor 66 and the flow control housing 64. In order to prevent the leakage, the flow control rotor 66 and the flow control housing 64 can be made of a material that can prevent a fluid from passing through the contact surface such as polymer, plastic, metallic substance, ABS, acrylic, or the like.

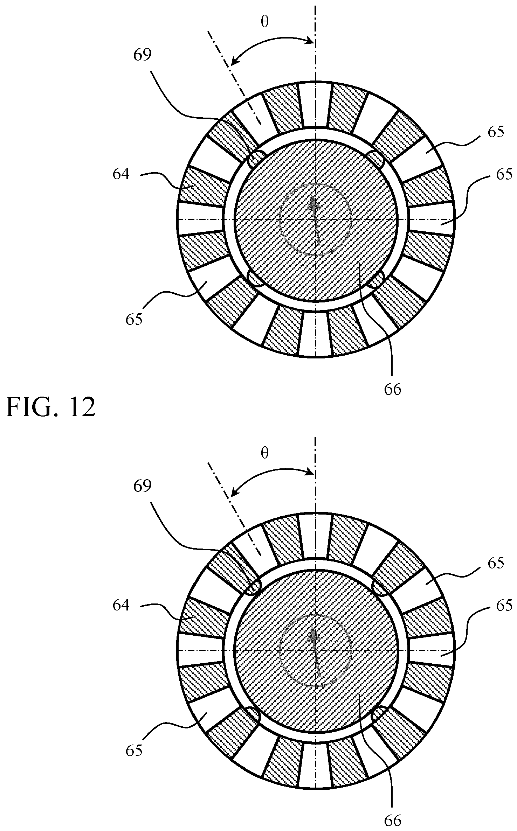

In addition, in order to prevent a leakage of fluid through the contact surface, the flow control rotor 66 may be provided with a protrusion 69 such as an o-ring or a gasket, as shown in FIG. 12. The protrusion 69 can be made of a flexible material such as rubber, poly er, silicone and the like, or an inflexible material such as metal, aluminum, plastic, polymer, and the like to efficiently prevent the fluid leakage. Alternatively, a protrusion 69 may be formed on the inner surface of the flow control housing 64.

The rotating type valve is not limited to the structure shown in the drawings and may be modified into different structures. In addition, the flow control unit 60 is not limited to the structures described above and may be modified into other structures that control a flow through the in-flow and out-flow tubes.

The blood processing apparatus 1 may also include various safety and monitoring sensors 24 and 34. The sensors monitor the blood processing treatment and may include pressure sensors, air bubble sensor, blood leak sensor, temperature sensor, a conductivity sensor, and the like. In addition, an additional filter such as an endotoxin filter may be installed in the circuit of the blood processing apparatus 1 to ensure no harmful substances to come in contact with blood.

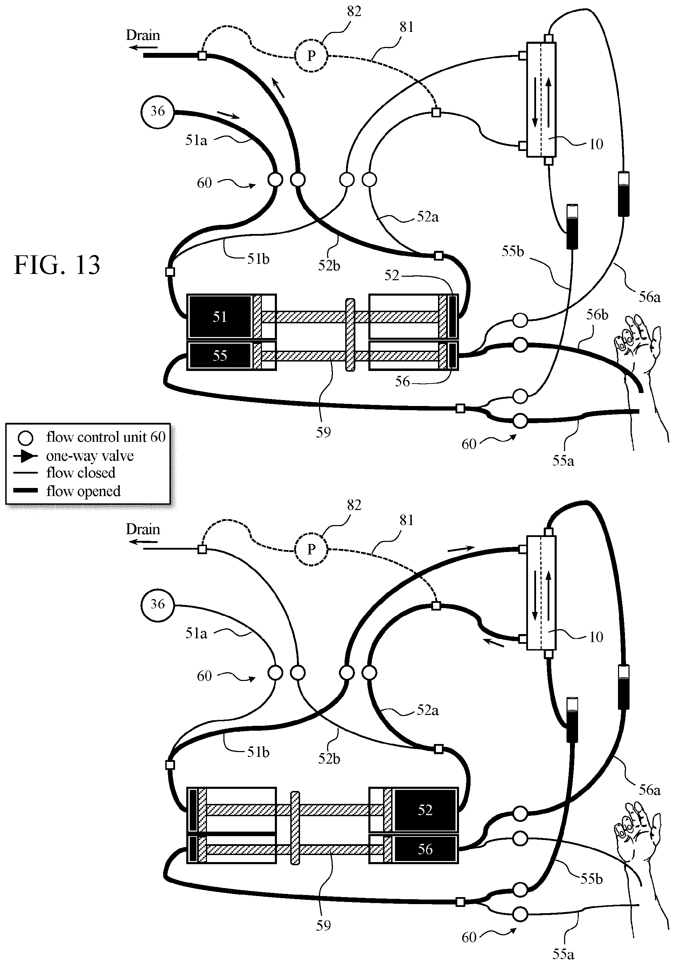

Hereinafter, embodiments of the blood processing apparatus 1 according to the present invention and their operations will be described in detail with reference to the accompanying drawings. FIGS. 13 to 28 are views illustrating the embodiments of the blood processing apparatus 1 and their operations.

### Example 1

The blood processing apparatus 1 includes four fluid chambers, i.e., the first, second, fifth, and sixth chambers 51, 52, 55 and 56 (FIG. 13). Two chambers 51 and 52 are connected to the dialysis fluid ports to transfer dialysis fluid through the blood processing filter 10 and the other two chambers 55 and 56 are connected to blood ports to transfer blood. The flow control unit 60 may be formed of a pressurizing type valve for the in-flow and out-flow tubes connected to the chambers.

When the second and sixth chambers 52 and 56 are compressed and the first and fifth chambers 51 and 55 are expanded, the flow control unit 60 opens a flow through the tubes 51a, 52b, 55a and 56b and blocks a flow through the tubes 51b, 52a, 55b and 56a (FIG. 13).

Due to the expansion of the first chamber 51, dialysis fluid is supplied to the chamber through the first chamber in-flow tube 51a. Due to the compression of the second chamber 52, dialysis fluid of the chamber is discarded therefrom through the second chamber out-flow tube 52b. Due to the expansion of the fifth chamber 55, blood of a patient is supplied to the chamber through the fifth chamber in-flow tube 55a. Due to the compression of the sixth chamber 56, blood of the chamber is returned to a patient. During this phase, neither blood nor dialysis fluid flows through the blood processing filter 10.

Here, thick black lines in the drawings represent that there is a flow therethrough, i.e., the flow control unit 60 opens a flow through the tube. Thin black lines represent that there is no flow therethrough, i.e., the flow control unit 60 blocks a flow through the tube. A dotted line represents an auxiliary dialysis fluid tube 81 and an auxiliary dialysis fluid pump 82.

On the other hand, when the chambers 52 and 56 are expanded and the chambers 51 and 55 are compressed, the flow control unit 60 blocks a flow through the tubes 51a, 52b, 55a and 56b and opens a flow through the tubes 51b, 52a, 55b and 56a.

Due to the compression of the chamber 51, dialysis fluid of the chamber is supplied to the blood processing filter 10 through the first chamber out-flow tube 51b. Due to the expansion of the chamber 52, dialysis fluid of the blood processing filter 10 is discharged to the chamber 52 through the second chamber in-flow tube 52a. Due to the compression of the chamber 55, blood is supplied to the blood processing filter 10 through the fifth chamber out-flow tube 55b. Due to the expansion of the chamber 56, blood of the blood processing filter 10 flows into the chamber 56. During this phase, blood and dialysis fluid both flow through the blood processing filter 10.

The chamber 51 serves as a means of providing fresh dialysis fluid to the blood processing filter 10 and the chamber 52 discharges used dialysis fluid from the blood processing filter 10. The chambers 55 provides blood of a patient to the blood processing filter 10 and the chamber 56 allows blood of the blood processing filter 10 to be returned to a patient. Since the dialysis fluid processing unit 30 produces dialysis fluid, the dialysis fluid processing unit 30 may be connected to the first chambers 51 through the first chamber in-flow tube 51.

The chambers 51, 52, 55 and 56 may have a predetermined stroke volume. The stroke volume of the chamber can be defined as a volume that is expanded or compressed when the chamber pressurizing member 59 expands or compresses the chamber. The chambers 51 and 52 may have substantially the same stroke volume as each other, and the chambers 55 and 56 may have substantially the same stroke volume as each other. In addition, the chambers 51 and 52 may have the stroke volume that is larger than that of the chambers 55 and 56. In an embodiment, the stroke volume of the chambers 51 and 52 may be approximately twice of the stroke volume of the chambers 55 and 56. However, the chamber's stoke volumes are not limited thereto and can be modified such that the chambers 55 and 56 have same stroke volumes that are different from each other and the chambers 51 and 52 have different stroke volumes from each other.

In order for the chambers to have substantially the same stroke volume, the cross-sectional surface area of the internal space of the chamber may be same as or substantially similar to each other. When the internal space of the chamber is shaped into a cylinder, the cross-sectional surface area depends on a diameter of the cross-sectional surface.

The blood processing apparatus 1 may be embodied into a structure, in which used dialysis fluid is discharged from the blood processing apparatus 1 through the chamber 51 and fresh dialysis fluid is provided to the blood processing filter 10 through the chamber 52. Similarly, blood of a patient is supplied to the blood processing filter 10 through the chamber 56 and blood of the blood processing filter 10 is returned to a patient through the chamber 55.

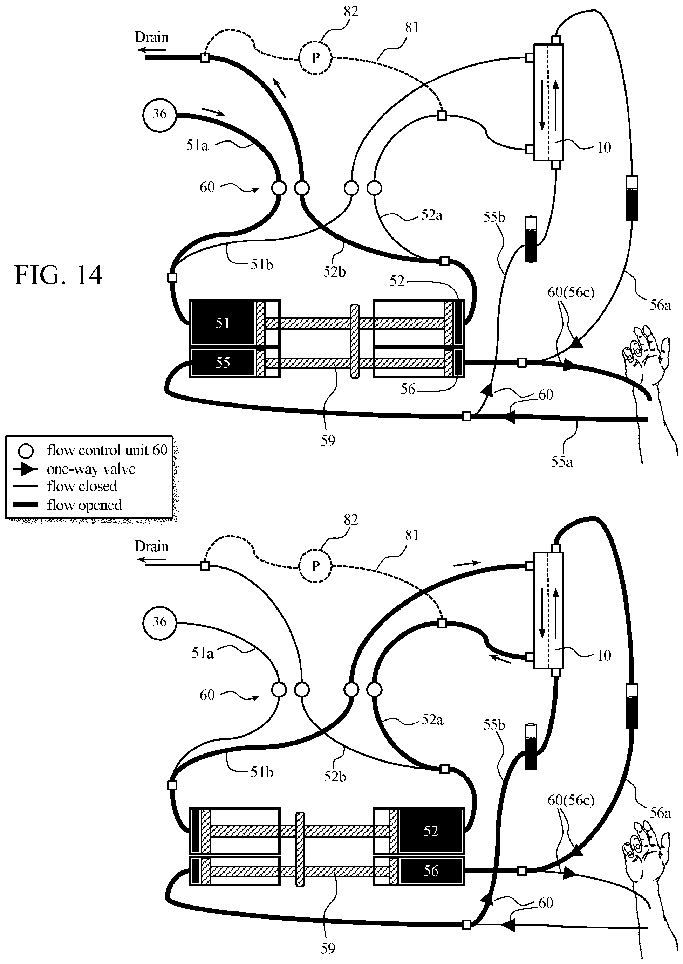

Furthermore, as shown in FIG. 14, the flow control unit 60 may be formed of a pressurizing type valve for the in-flow and out-flow tubes connected to the chambers 51 and 52 but it is formed of one-way check valves 55c and 56c for the tubes connected to the chambers 55 and 56 to control a flow therethrough.

### Example 2

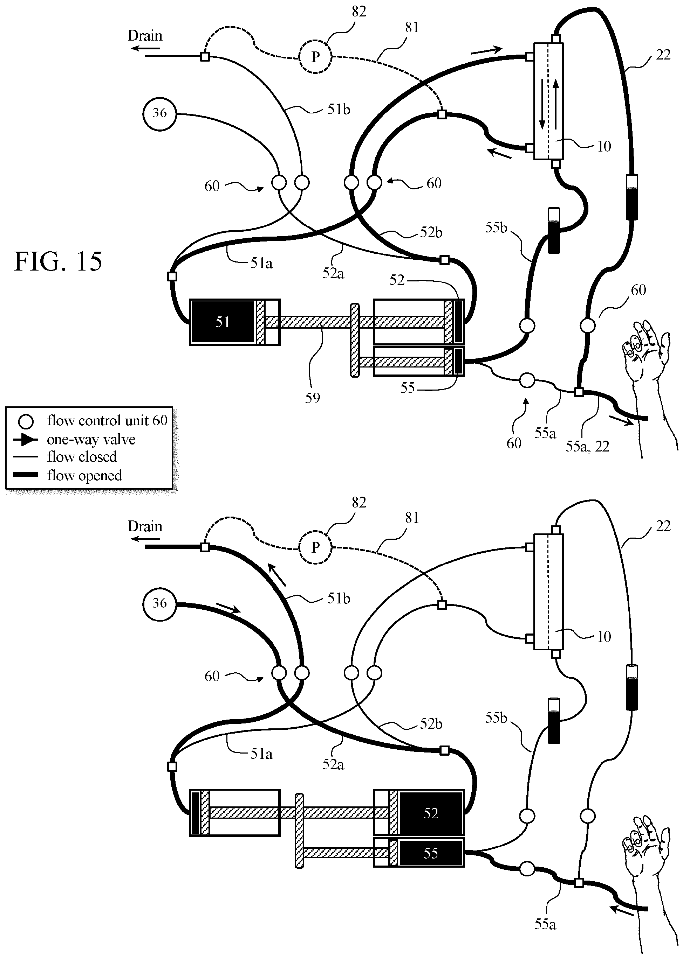

The blood processing apparatus 1 is formed with three fluid chambers including a first chamber 51, a second chamber 52, and a fifth chamber 55 (FIG. 15). Two chambers 51 and 52 are connected to the dialysis fluid ports to transfer dialysis fluid and one chamber 55 is connected to the blood port to transfer blood. The flow control unit 60 controls a flow through the tubes connected to the chambers 51, 52 and 55, and may be formed of a pressurizing type valve. Here, an additional flow control unit 60 may be provided in the blood tube 22 connected to the second blood port 14 to open or close the flow therethrough.

When the chambers 52 and 55 are compressed and the chamber 51 is expanded, the flow control unit 60 opens a flow through the tubes 51a, 52b, 55b and 22, and blocks a flow through the tubes 51b, 52a and 55a (FIG. 15). Due to the expansion of the chamber 51, dialysis fluid of the blood processing filter 10 flows into the chamber through the first chamber in-flow tube 51a. Due to the compression of the chamber 52, the dialysis fluid of the chamber is supplied to the blood processing filter 10 through the second chamber out-flow tube 52b. Due to the compression of the chamber 55, blood inside the chamber is supplied to the blood processing filter 10 through the fifth chamber out-flow tube 55b and returned to a patient through the blood tube 22.

When the chambers 52 and 55 are expanded and the chamber 51 is compressed, the flow control unit 60 blocks a flow through the tubes 51a, 52b, 55b and 22, and opens a flow through the tubes 51b, 52a and 55a (FIG. 15). Due to the compression of the chamber 51, dialysis fluid of the chamber is discarded through the first chamber out-flow tube 51b. Due to the expansion of the chamber 52, dialysis fluid is furnished to the chamber through the second chamber in-flow tube 52a. Due to the expansion of the chamber 55, blood of a patient is supplied to the chamber.

As shown in the drawing, blood may be withdrawn from a patient and returned back to a patient through a single needle access connected to a patient.

### Example 3

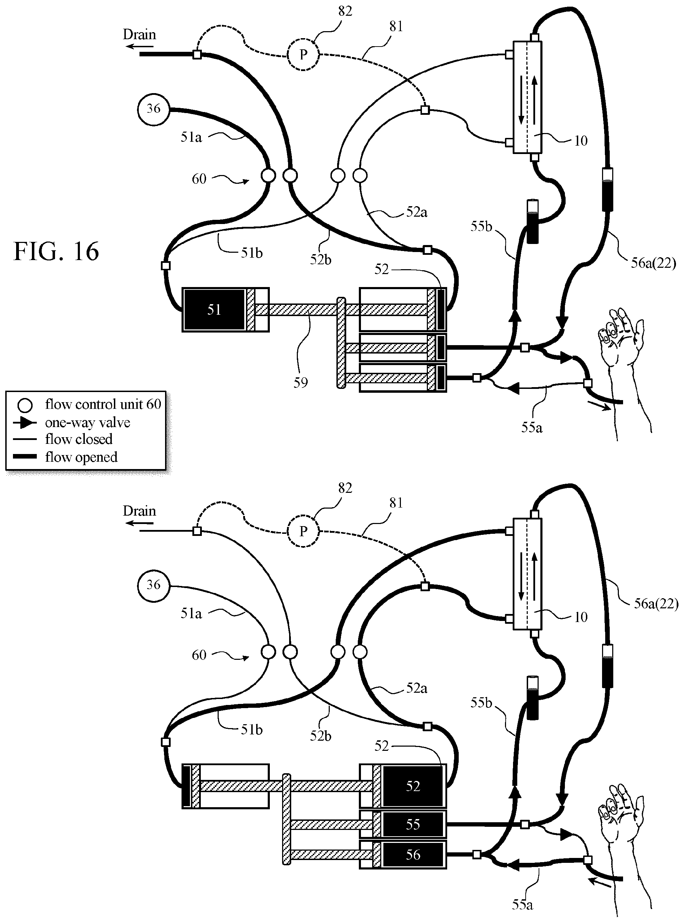

The blood processing apparatus 1 may be modified into a structure where blood of a patient is supplied to the blood processing apparatus 1 and returned to a patient by using two chambers 55 and 56, but these two separate chambers 55 and 56 are simultaneously compressed or expanded (FIG. 16). The blood processing apparatus 1 includes four fluid chambers 51, 52, 55 and 56, but unlike Example 1, one chamber is compressed while three chambers are expanded, and one chamber is expanded while three chambers are compressed.

The flow control unit 60 for the tubes 55a, 55b, 56a, and 56b connected to chambers 55 and 56 may be formed of the one-way valves. Despite a similar operation with Example 2, the blood processing apparatus 1 of FIG. 16 may be able to allow blood to flow through the blood processing filter 10 when the chambers 55 and 56 are both compressed and expanded.

### Example 4

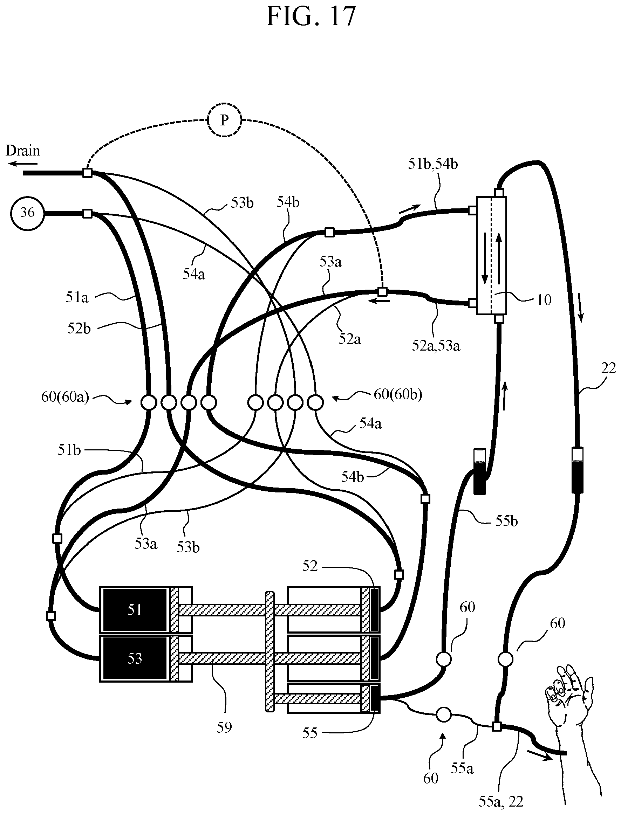

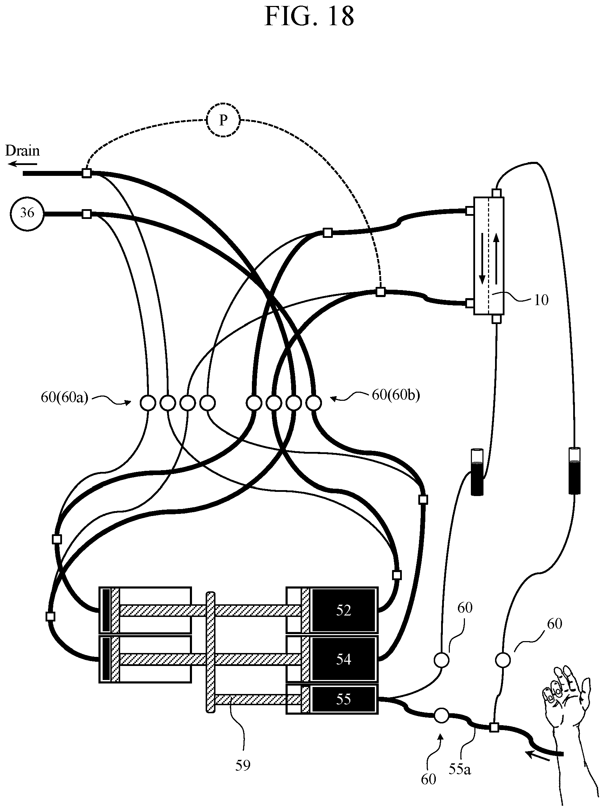

FIGS. 17 and 18 illustrate flow circuit diagrams of the blood processing apparatus 1 having five (5) fluid chambers. Specifically, four chambers 51 to 54 are connected to the dialysis fluid port 15 or 16 and circulate dialysis fluid through the blood processing filter 10, and a single chamber 55 is connected to the blood port 13 to transfer blood.

Blood is transferred to the blood processing filter 10 when the chamber pressurizing member 59 moves to a right direction in FIG. 17, i.e., compressing the chambers 52, 54 and 55. Since the four chambers are used to transfer dialysis fluid, there is a continuous flow of the dialysis fluid through the blood processing filter 10 when the chambers are compressed and expanded.

The dialysis fluid circuit and the operation are similar to Examples 5 and 6 which are described below in more detail, whereas the blood circuit and the operation are similar to Example 2.

In a similar manner, the blood processing apparatus 1 according to an embodiment of the present invention may be modified into a structure where six fluid chambers 51 to 56 are used for transferring blood and dialysis fluid. Dialysis fluid is transferred through four chambers 51 to 54, as show in FIGS. 17 and 18. However, blood is transferred using two chambers 55 and 56 but the two chambers 55 and 56 are simultaneously compressed (or expanded), similar to that shown in FIG. 16. The flow control unit 60 may be formed of one-way valves for the tubes connected to the chambers 55 and 56, i.e., the tubes 55a, 55b, 56a and 56b. The flow control unit 60 for the tubes connected to the chambers 51 to 54 may be any one, or a combination, of the one-way valves, solenoid valves, pressurizing type valves, or the rotating-type valves.

### Example 5

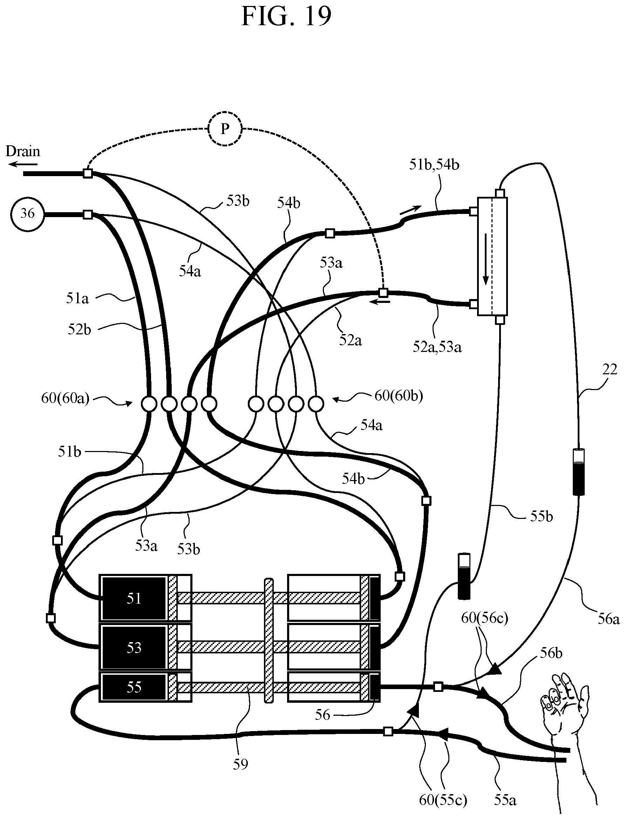

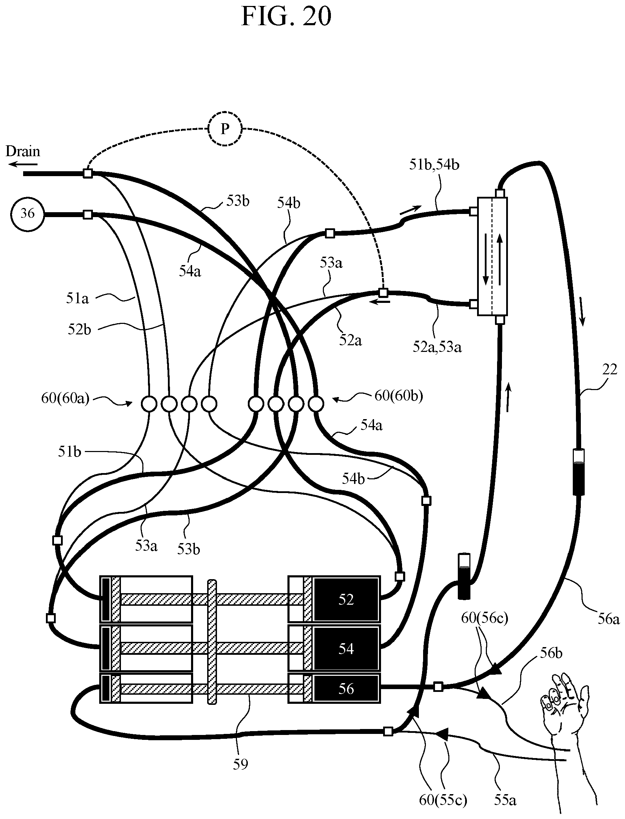

FIGS. 19 and 20 are views illustrating the blood processing apparatus 1 according to another embodiment of the present invention. The blood processing apparatus 1 includes six fluid chambers, i.e., first to sixth chambers 51 to 56. Dialysis fluid flows through the first to fourth chambers 51 to 54. Specifically, dialysis fluid is supplied to the blood processing filter 10 through the first and fourth chambers 51 and 54, and is removed from the blood processing filter 10 through the second and third chamber 52 and 53. Thus, the chambers 51 and 54 are connected to the first dialysis fluid port 15 and the chambers 52 and 53 are connected to the second dialysis fluid port 16. The dialysis fluid processing unit 30 may be connected to the first and fourth chambers 51 and 54 (through the first and fourth chamber in-flow tubes 51a and 54a).

Blood flows through the chambers 55 and 56. Blood is supplied to the blood processing filter 10 through the fifth chamber 55 and blood of the blood processing filter 10 is returned to a patient through the sixth chamber 56. Thus, the fifth chamber 55 is connected to the first blood port 13 and the sixth chamber 56 is connected to the second blood port 14.

The flow control unit 60 is formed of the pressurizing type valve for the tubes connected to the chambers 51 to 54 and one-way valves for the tubes connected to the chambers 55 and 56.

The chambers 51, 53, and 55 are expanded and the chambers 52, 54 and 56 are compressed (FIG. 19). The flow control unit 60 opens a flow through the tubes 51a, 52b, 53a and 54b, and blocks a flow through the tubes 51b, 52a, 53b and 54a.

Due to the expansion of the first chamber 51, dialysis fluid is supplied to the chamber through the first chamber in-flow tube 51a. Due to the compression of the second chamber 52, dialysis fluid of the chamber is discharged therefrom through the second chamber out-flow tube 52b. Due to the expansion of the third chamber 53, dialysis fluid of the blood processing filter 10 is supplied to the chamber through the third chamber in-flow tube 53a. Due to the compression of the fourth chamber 54, dialysis fluid of the chamber is supplied to the blood processing filter 10 through the fourth chamber out-flow tube 54b. Due to the expansion of the fifth chamber 55, blood of a patient flows into the chamber through the fifth chamber in-flow tube 55a. Here, due to the one-way valve 55c, blood of the blood processing filter 10 may not flow backward to the chamber 55. Finally, due to the compression of the sixth chamber 56, blood of the chamber is returned to a patient through the sixth chamber out-flow tube 56b. Blood in the chamber 56 may not flow backward to the blood processing filter 10 because of the one-way valve 56c. During this phase, no blood flows through the blood processing filter 10.

On the other hand, the chambers 51, 53 and 55 are compressed while the chambers 52, 54 and 56 are expanded (FIG. 20). The flow control unit 60 blocks a flow through the tubes 51a, 52b, 53a, 54b, and opens a flow through the tubes 51b, 52a, 53b, 54a.

Due to the compression of the first chamber 51, dialysis fluid of the chamber is provided to the blood processing filter 10 through the first chamber out-flow tube 51b. Due to the expansion of the second chamber 52, dialysis fluid of the blood processing filter 10 is discharged to the chamber 52 through the second chamber in-flow tube 52a. Due to the compression of the third chamber 53, dialysis fluid of the chamber is removed therefrom through the third chamber out-flow tube 53b. Due to the expansion of the fourth chamber 54, dialysis fluid is supplied to the chamber 54 through the fourth chamber in-flow tube 54a. Due to the compression of the fifth chamber 55, blood of the chamber is supplied to the blood processing filter 10 through the fifth chamber out-flow tube 55b. Here, blood of the chamber 55 may not flow backward to a patient because of the one-way valve 55c. Finally, due to the expansion of the sixth chamber 56, blood of the blood processing filter 10 flows into the sixth chamber 56 through the sixth chamber in-flow tube 56a. Patient's blood may not flow into the chamber 56 due to the one-way valve 56c. Thus, during this phase, both blood and dialysis fluid flow through the blood processing filter 10.

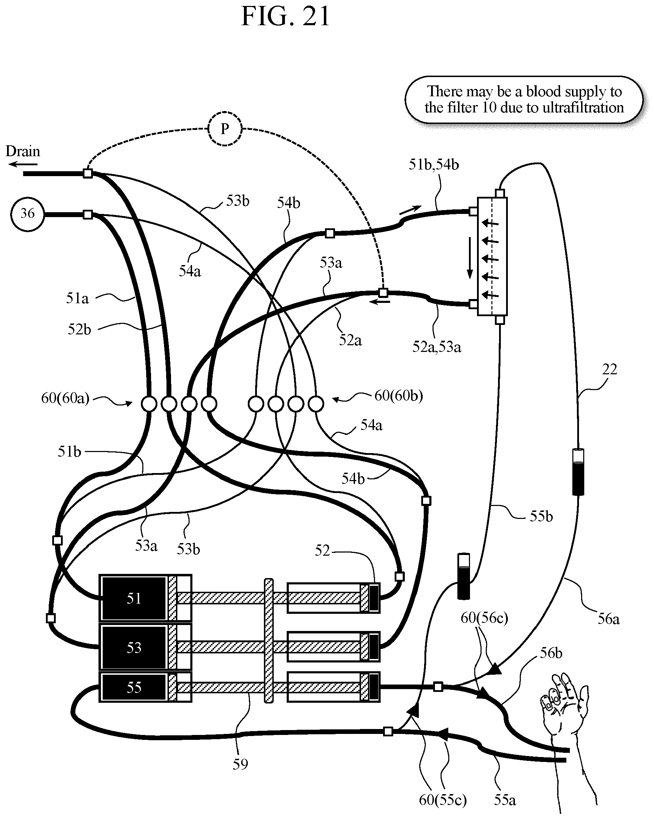

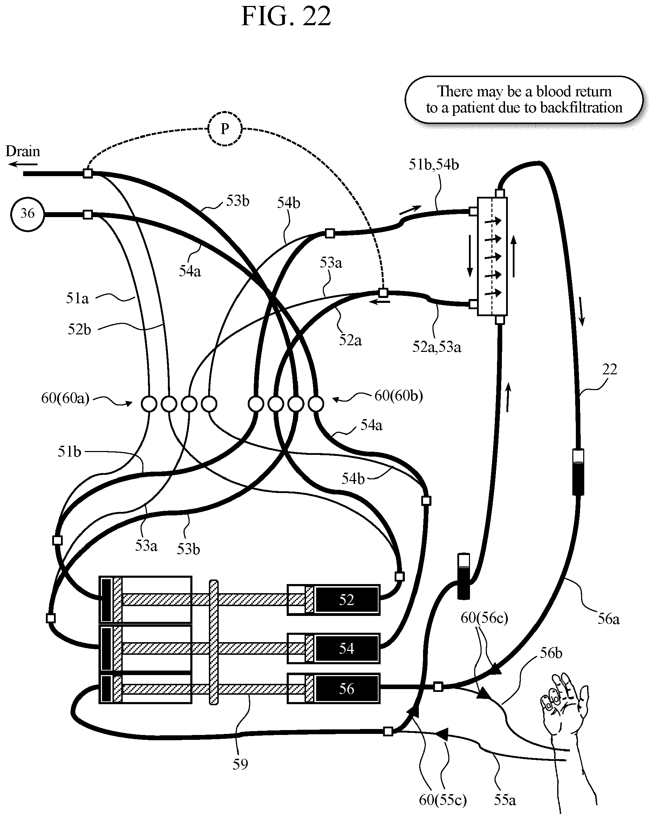

As mentioned above, the chambers 51 to 56 may have stroke volumes that are different from each other. For example, as shown in FIGS. 21 and 22, the chambers 51 and 53 may have a stroke volume that is larger than that of the chambers 52 and 54. When the chamber pressurizing member 59 moves left (FIG. 21), since the third chamber 53 has a larger stroke volume than the fourth chamber 54, ultrafiltration occurs in which plasma water of the blood moves to the dialysis fluid compartment across the blood processing membrane 12.

On the contrary, when the chamber pressurizing member 59 moves right (FIG. 22), since the first chamber 51 has a larger stroke volume than the second chamber 52, backfiltration in which dialysis fluid moves to the blood compartment of the blood processing filter 10 occurs. Thus, the stroke volumes of the chambers can regulate the ultrafiltration and backfiltration rates, which results in dynamic mass transfer between blood and dialysis fluid.

In a similar manner, the stoke volumes of the chamber 55 and 56 may be modified to be equal to each other or to be different from each other, which is aimed at regulating the net filtration (i.e., a difference of ultrafiltration and backfiltration). Otherwise, the stroke volumes of the six chambers may be substantially similar to or same as each other.

The flow control unit 60 is formed of the pressurizing type valve for the tubes connected to the first to fourth chambers 51 to 54 and one-way valves for the tubes connected to the fifth and sixth chambers 55 and 56. However, the flow control unit 60 is not limited thereto and may be modified. For example, the flow control unit 60 can be formed of the pressurizing type valves to open or close the flow through the tubes connected to all of the first to sixth chambers 51 to 56, i.e., the tubes 51a, 51b, 52a, 52b, 53a, 53b, 54a, 54b, 55a, 55b, 56a, and 56b, as shown in FIG. 8. Alternatively, the flow control unit 60 may be formed of one-way valves installed in each of the tubes connected to the first to sixth chambers 51 to 56, which is described below.

### Example 6

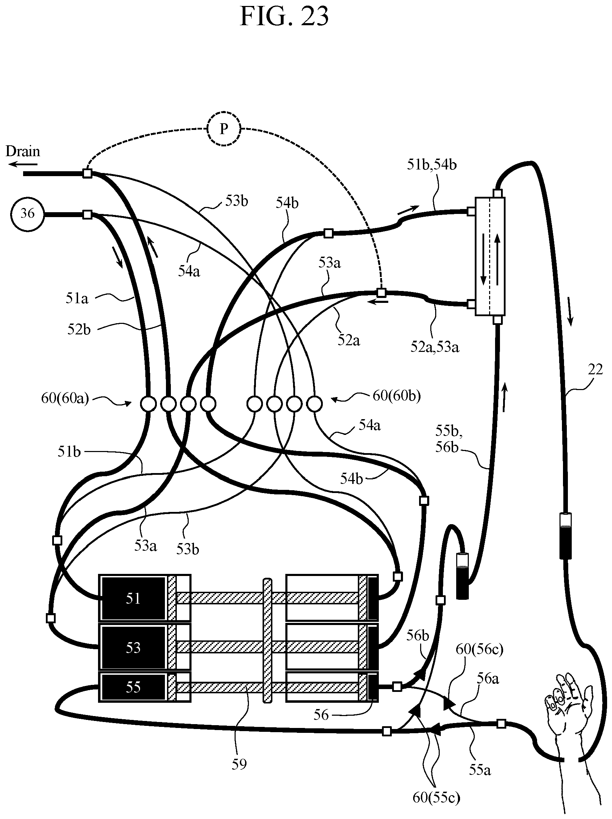

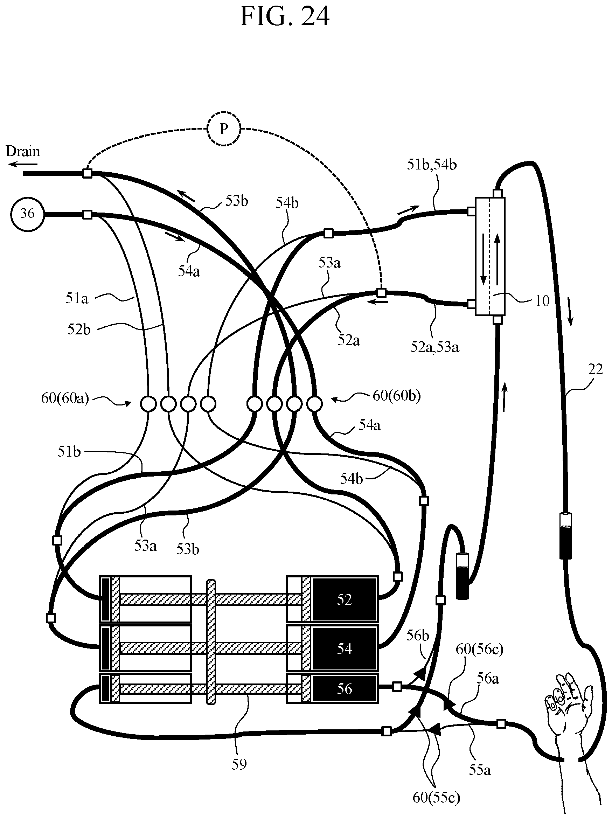

FIGS. 23 and 24 are views illustrating the blood processing apparatus 1, in which blood is supplied to the blood processing apparatus 1 through the fifth and sixth chambers 55 and 56. But unlike Example 5, the fifth and sixth chambers 55 and 56 are both connected to the first blood port 13.

When the chambers 51, 53 and 55 are expanded and the chambers 52, 54 and 56 are compressed (FIG. 23), due to the expansion of the fifth chamber 55, blood of a patient is supplied to the chamber through the fifth chamber in-flow tube 55a. At the same time, due to the compression of the sixth chamber 56, blood of the chamber is supplied to the blood processing filter 10 through the sixth chamber out-flow tube 56b.

On the other hand, when the chambers 51, 53 and 55 are compressed and the chambers 52, 54 and 56 are expanded (FIG. 24), due to the compression of the fifth chamber 55, blood of the chamber is supplied to the blood processing filter 10 through the fifth chamber out-flow tube 55b. Simultaneously, the expansion of the sixth chamber 56 allows blood of a patient to be supplied to the sixth chamber 56 through the sixth chamber in-flow tube 56a. Thus, both blood and dialysis fluid continuously flow through the blood processing filter 10 while the chambers are compressed or expanded.

Once again, the chambers 51 to 54 may have substantially the same stroke volume as each other, but the chambers 55 and 56 may have a different stroke volume from that of the chambers 51 to 54. For example, the stroke volume of the chambers 55 and 56 may have approximately a half of the stroke volume of the chambers 51 to 54.

In addition, among the first to sixth chambers 51 to 56, the number of chambers through which a first fluid flows and the number of chambers through which a second fluid flows may be changed depending on the objective of the blood processing treatment.

### Example 7

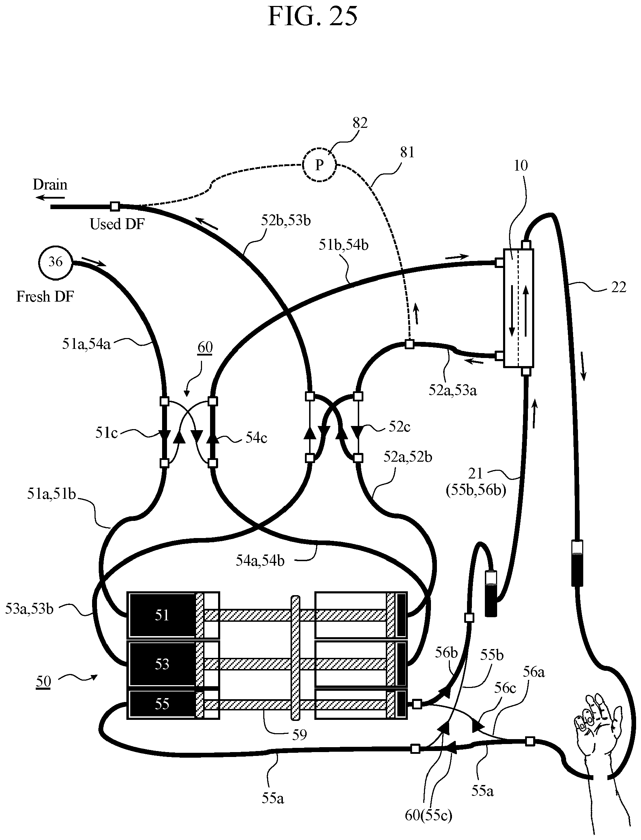

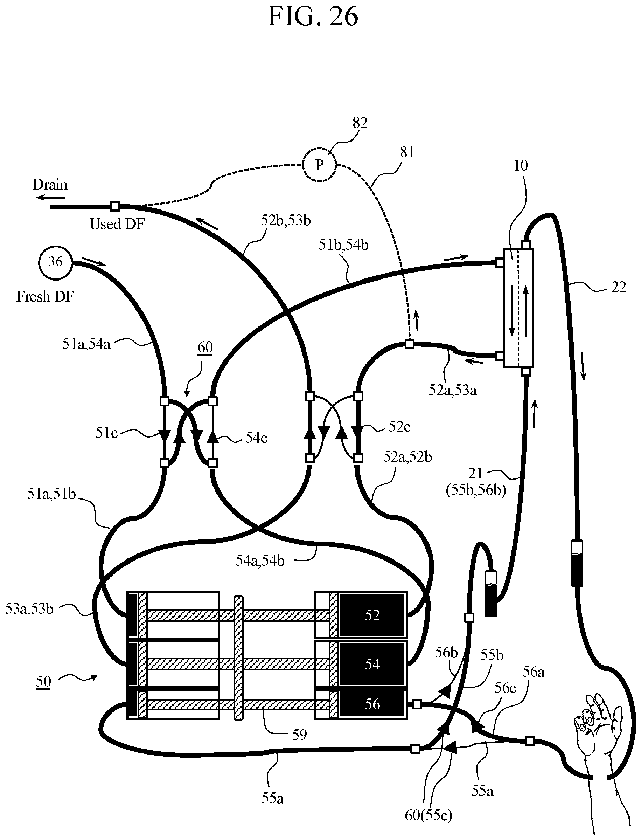

FIGS. 25 and 26 are views illustrating the blood processing apparatus 1 according to another embodiment of the present invention, in which the flow control unit 60 is formed of one-way valves installed on each of the flow tubes connected to the first to sixth chambers 51 to 56. First chamber one-way valves 51c are installed on the first chamber in-flow and out-flow tubes 51a and 51b. Second chamber one-way valves 52c are installed on the second chamber in-flow and out-flow tubes 52a and 52b.

The chambers 51, 53 and 55 are expanded and the chambers 52, 54 and 56 are compressed (FIG. 25).

Due to the expansion of the first chamber 51, dialysis fluid is supplied to the chamber through the first chamber in-flow tube 51a. Here, because of the one-way valve 51c, dialysis fluid of the blood processing filter 10 may not flow backward to the chamber. Due to the compression of the second chamber 52, dialysis fluid of the chamber is discharged therefrom through the second chamber out-flow tube 52b. Because of the one-way valve 52c, dialysis fluid may not flow backward to the blood processing filter 10. Due to the expansion of the third chamber 53, dialysis fluid of the blood processing filter 10 is supplied to the chamber through the third chamber in-flow tube 53a. Here, because of the one-way valve 53c, used dialysis fluid may not flow backward to the chamber. Due to the compression of the fourth chamber 54, dialysis fluid of the chamber is supplied to the blood processing filter 10 through the fourth chamber out-flow tube 54b. Because of the one-way valve 54c, dialysis fluid may not flow backward to the fresh dialysis fluid container 36. Due to the expansion of the fifth chamber 55, blood of a patient is supplied to the chamber through the fifth chamber in-flow tube 55a. Because of the check valve 55c, blood of the blood processing filter 10 may not flow backward to the chamber. Due to the compression of the sixth chamber 56, blood of the chamber is supplied to the blood processing filter 10 through the sixth chamber out-flow tube 56b. Due to the one-way valve 56c, blood of the chamber may not flow backward to a patient.

On the other hand, the chambers 51, 53 and 55 are compressed and the chambers 52, 54 and 56 are expanded (FIG. 26).

Due to the compression of the first chamber 51, dialysis fluid of the chamber is supplied to the blood processing filter 10 through the first chamber out-flow tube 51b. Because of the one-way valve 51c, dialysis fluid may not flow backward to the fresh dialysis fluid container 36 (i.e., through the first chamber in-flow tube 51a). Due to the expansion of the second chamber 52, dialysis fluid of the blood processing filter 10 is supplied to the chamber 52 through the second chamber in-flow tube 52a. Because of the one-way valve 52c, used dialysis fluid may not flow backward to the second chamber. Due to the compression of the third chamber 53, dialysis fluid of the chamber is removed through the third chamber out-flow tube 53b. Because of the one-way valve 53c, dialysis fluid may not flow backward to the blood processing filter 10. Due to the expansion of the fourth chamber 54, dialysis fluid is supplied to the chamber 54 through the fourth chamber in-flow tube 54a. Because of the one-way valve 54c, dialysis fluid of the blood processing filter 10 may not flow backward to the chamber 54. Due to the compression of the fifth chamber 55, blood of the chamber is supplied to the blood processing filter 10 through the fifth chamber out-flow tube 55b. Due to the one-way valve 55c, blood of the chamber may not flow backward to a patient. Due to the expansion of the sixth chamber 56, blood of a patient is supplied to the sixth chamber 56 through the sixth chamber in-flow tube 56a. Because of the check valve 56c, blood of the blood processing filter 10 may not flow backward to the chamber.

The one-way valves installed on the corresponding flow tubes enforce a fluid to flow in one direction, preventing a retrograde flow through the tubes. Therefore, the one-way valve according to an embodiment of the present invention may have a predetermined cracking pressure. The cracking pressure is the pressure difference between upstream and downstream of the one-way valve which opens a flow though the valve. For example, when the flow control unit 60 is not operating, the one-way valve may have a cracking pressure value which is enough to prevent a fluid from flowing through the one-way valves. In an embodiment, the check valve may have the cracking pressure of 10 mmHg to 180 mmHg, more preferably 12 mmHg to 60 mmHg.

### Example 8

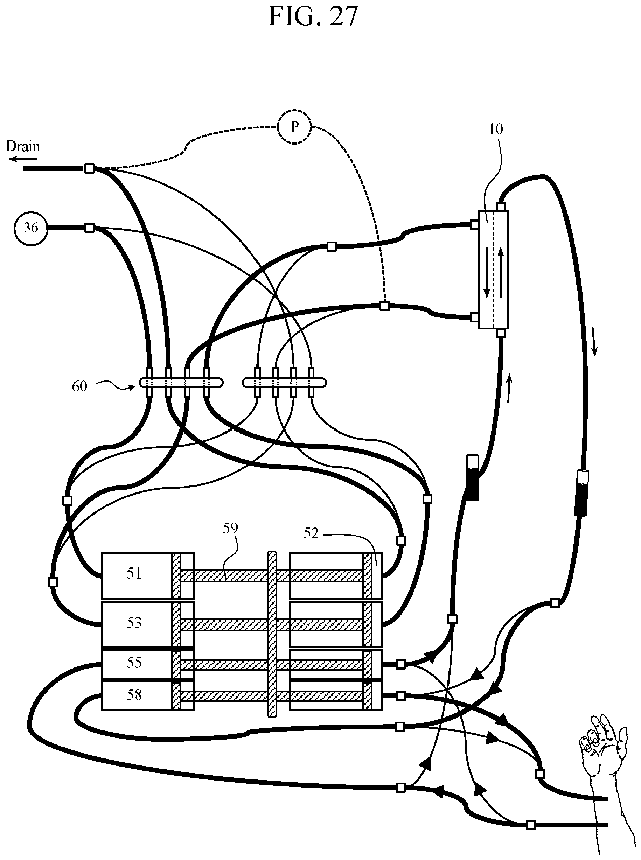

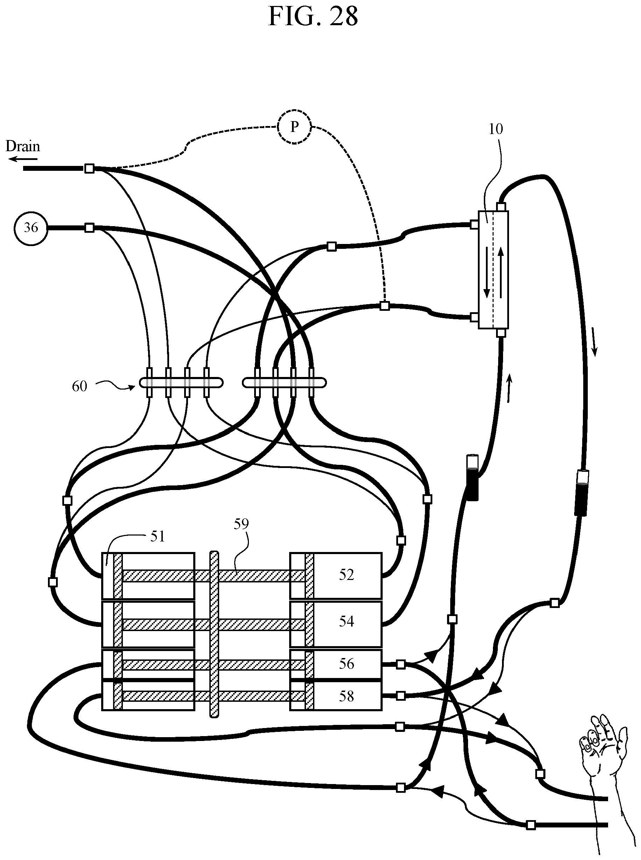

The blood processing apparatus 1 according to an embodiment of the present invention may be embodied into other structures. For example, as shown in FIGS. 27 and 28, the blood processing apparatus 1 is formed of eight (8) fluid chambers. Four chambers transfer dialysis fluid and the other four chambers transfer blood.

Dialysis fluid is transferred by the operation of the four chambers 51 to 54. Specifically, two chambers 51 and 54 supply dialysis fluid to the blood processing filter 10 and another two chambers 52 and 53 remove the dialysis fluid from the blood processing filter 10. Detailed description of the operation of the four chambers are described above.

Blood is also transferred by the operation of the four chambers 55 to 58. Two chambers supply blood to the blood processing filter 10 and the other two chambers return blood of the blood processing filter 10 to a patient. Detailed description of the operation of the four chambers are also described above.

The flow control unit 60 is configured to control a flow through the tubes connected to the chambers 51 to 58, comprising any one or a combination of one-way valves, solenoid valves, on-off valves, the pressurizing type valve, or the rotating type valve. The flow control unit 60 may be able to block eight (8) tubes while opening the other eight (8) tubes, or vice versa.

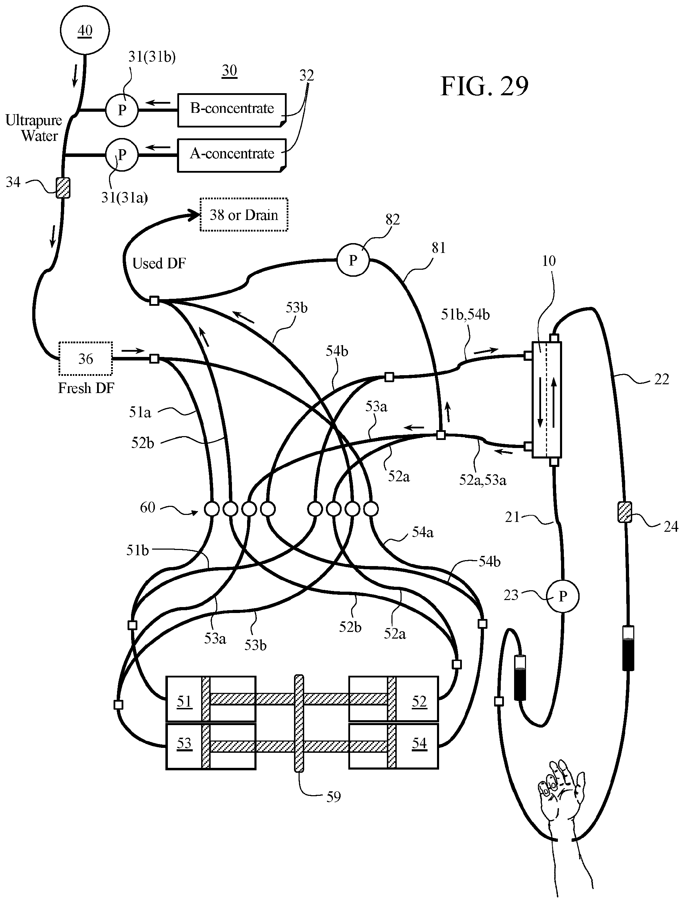

The blood processing apparatus 1 according to an embodiment of the present invention may further be embodied such that a blood pump 23 is provided in the blood tube 21 or 22 to transfer blood. FIG. 29 is a view illustrating the blood processing apparatus 1 in which the blood pump 23 is installed in the blood tube 21 to supply blood to the blood processing filter 10. Dialysis fluid is transferred by the four chambers 51 to 54. Two chambers 51 and 54 supply dialysis fluid to the blood processing filter 10 while two chambers 52 and 53 remove the dialysis fluid from the blood processing filter 10.

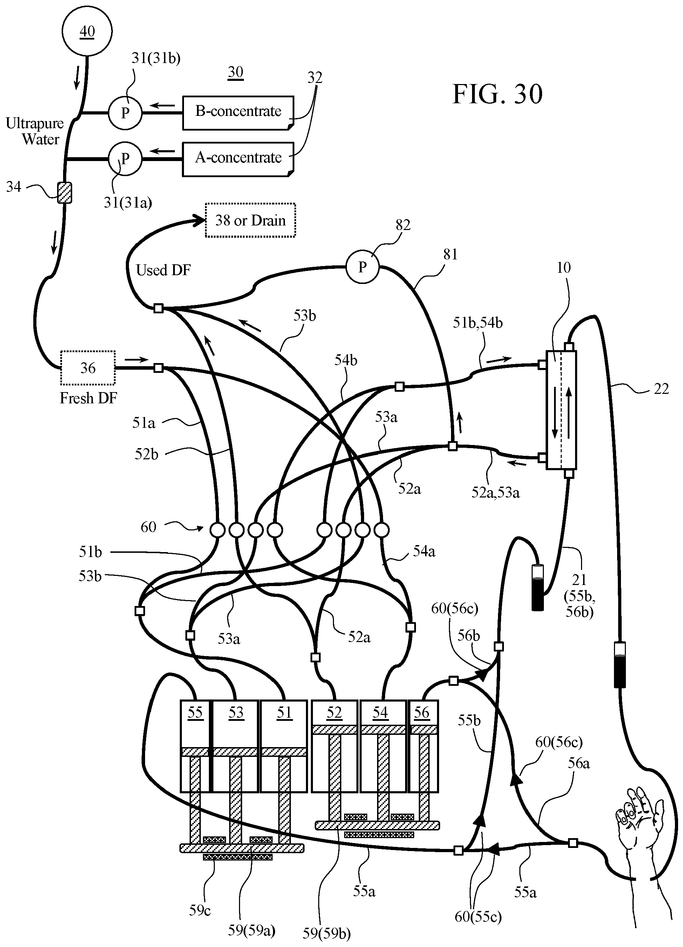

In addition, as illustrated in FIG. 30, the plurality of fluid chambers may be disposed in a vertical direction. A half of the chambers (at the left side) may be compressed while another half of the chambers (in the right side) is expanded, or vice versa. Also, the chamber pressurizing member 59 may include a first chamber pressurizing member 59a and a second chamber pressurizing member 59b, expanding and compressing the chambers in the left and right sides, respectively, in the drawings. The pressurizing type valve are depicted as the flow control unit 60 for the tubes connected to the chambers 51 to 54 and one-way valves are used for the tubes connected to the chambers 55 and 56. But, the flow control unit 60 may be modified to the pressurizing type valve, the rotating type valve, solenoid valves, or one-way valves, or a combination thereof to control a flow passage through the tubes connected to the chambers 51 to 56.

Furthermore, the blood processing apparatus 1 may further be provided with the auxiliary dialysis fluid tube 81 and the auxiliary dialysis fluid pump 82. The auxiliary dialysis fluid pump 82 is installed on the auxiliary dialysis fluid tube 81 to additionally remove dialysis fluid from the blood processing filter 10. The auxiliary dialysis fluid tube 81 may connect between the in-flow tube of the chambers 52 and 53 (i.e., the outlet of the blood processing filter 10) and the used dialysis fluid container 38 (or a drain line).

While the fluid pumping unit 50 supplies a predetermined amount of dialysis fluid to the blood processing filter 10 and then removes substantially the same amount of the dialysis fluid from the blood processing filter 10, the auxiliary dialysis fluid pump 82 removes additional dialysis fluid from the blood processing filter 10. Thus, the auxiliary dialysis fluid pump 82 may determine the net volume removal from a patient. Since the auxiliary dialysis fluid pump 82 determines the patient hydration level, it is required that the auxiliary dialysis fluid pump 82 delivers an exact amount of dialysis fluid. The auxiliary dialysis fluid pump 82 includes a various metering fluid pump such as a peristaltic pump, a roller pump, a cylinder-based pulsatile pump, a gear pump and other fluid pumps. In an embodiment, a metering rotary piston pump may be used for the auxiliary dialysis fluid pump 82.

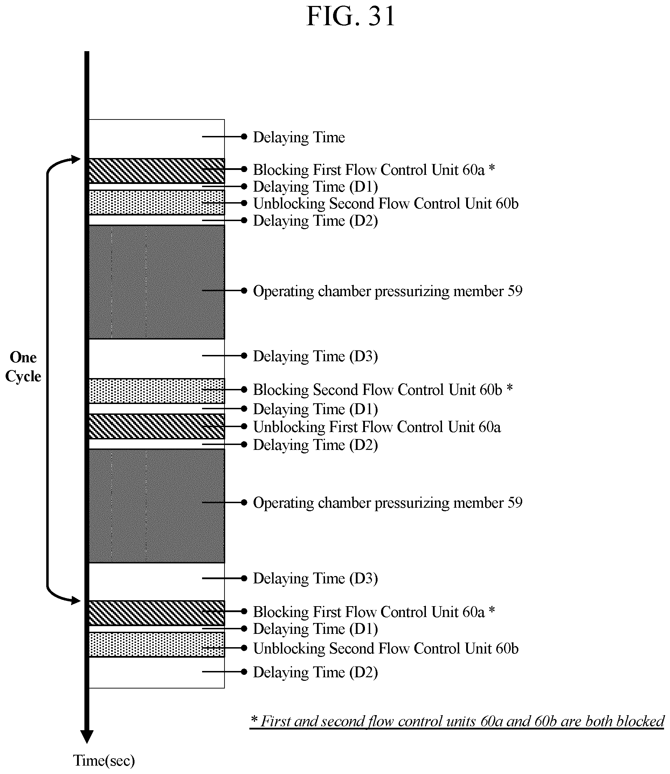

FIG. 31 is a view illustrating the flow control method of the flow control unit 60. The flow control unit 60 blocks a flow through some of the tubes and, at the same time, opens a flow through some other flow tubes. The flow control unit 60 repeats the blocking and the opening. The flow control unit 60 may include the first flow control unit 60a and the second flow control unit 60b, as shown in FIGS. 7 and 8. For example, the flow control unit 60 of Example 1 closes the flow passages through the tubes 51a, 52b, 55a, 56b and the tubes 51b, 52a, 55b, 56a in an alternate manner. Thus, the flow through tubes 51a, 52b, 55a, 56b may be controlled by the first flow control unit 60a and the flow through the tubes 51b, 52a, 55b, 56a may be controlled by the second flow control unit 60b. When the first flow control unit 60a blocks the flow, the second flow control unit 60b may open the flow, and vice versa.

Although the first flow control unit 60a and the second flow control unit 60b repeat the compression and expansion of the tubes, there is a moment when the first and second flow control units 60a and 60b both block the flow through the tubes. The flow control unit 60 may be configured to instantaneously close the flow through all of the tubes through which the flow control unit 60 controls a flow, such as when the first and second flow control units 60a and 60b switch the compression and expansion.

Thus, as shown in FIG. 31, the control method of the flow control unit 60 may include the steps of:
(S 1) blocking first flow control unit 60a,
(S2) unblocking second flow control unit 60b,
(S3) operating chamber pressurizing member 59,
(S4) blocking second flow control unit 60b,
(S5) unblocking first flow control unit 60a, and
(S6) operating chamber pressurizing member 59.

When the chamber pressurizing member 59 moves to one direction at S3, it moves to an opposite direction at S6, thereby allowing the chamber pressurizing member 59 to repeat the compression and expansion of the chambers. In addition, at S1 and S4, the first flow control unit 60a and second flow control unit 60b are both blocked; that is, the control units 60a and 60b both block the flow through the tubes. However, the first flow control unit 60a alone is blocked at S2 and the second flow control unit 60b is only blocked at S5.

The blood processing apparatus 1 may further include the steps of delaying time between steps, such as pausing for a predetermined of time. For example, there is a time delay (D1) between S1 and S2, there is a time delay (D2) between S2 and S3, and there is a time delay (D3) between S3 and S4. The D1, D2 and D3 have a predetermined value to improve the stability of the flow control operation. In an embodiment, D1 and D2 has a value between 0 and 1.2 sec, and D3 has a value from 0 to 2.5 sec.

In addition, S1 and S4 may take substantially the same period of time, and similarly, S2 and S5 take substantially the same time. Otherwise, S1, S2, S4, and S5 may take same time such as between 0.2 and 1.2 sec (preferably between 0.4 and 0.8 sec) while S3 and S6 take same time between 0.4 and 2.4 sec.

The blood processing apparatus according to an embodiment of the present invention compresses and expands multiple fluid chambers to transfer blood and dialysis fluid. The multiple chambers ensure the flow amount of the dialysis fluid upstream and downstream of the blood processing filter to be equally maintained, and neither a separate blood pump nor a balancing chamber may be required. Consequently, the entire blood processing system can be sufficiently miniaturized and light-weighted, and easy to be installed while reducing the cost for blood processing treatment. The blood processing apparatus will be suitable for an optimal alternative for the blood processing treatment in a place out of hospitals.

## Claims

1. A blood processing apparatus (1) comprising:
a plurality of fluid chambers each having an internal space;
a chamber pressurizing member (59) compressing or expanding the internal spaces of the fluid chambers (51, 52, 53, 54, 55, 56, 57, 58);
a chamber pressurizing member driver driving the chamber pressurizing member (59); and
a flow control unit (60), wherein
the plurality of fluid chambers (51, 52, 53, 54, 55, 56, 57, 58) includes n fluid chambers (where n is 2 or more and a positive integer),
the n fluid chambers are each connected with a first flow tube through which a fluid is provided to the chamber and a second flow tube through which a fluid of the chamber is discharged therefrom, and
the flow control unit (60) controls a flow through the first and second flow tubes connected to the n chambers and
**characterized in that** when the n is an even number, n/2 chambers are compressed and another n/2 chambers are expanded simultaneously,
wherein when the n is an odd number, (n+1)/2 chambers are compressed and (n-1)/2 chambers are expanded simultaneously.

2. The blood processing apparatus (1) of claim 1, further comprising a blood processing filter (10) in which blood is processed, wherein the blood processing filter (10) comprises:
a filter housing (11) having an internal space;
a first blood port (13) provided in an end of the filter housing (11) and through which blood is provided to the blood processing filter (10);
a second blood port (14) provided in another end of the filter housing (11) and through which blood is discharged from the blood processing filter (10); and
at least one dialysis fluid port (15) disposed at the filter housing (11) and through which dialysis fluid is provided to or discharged from the blood processing filter (10).

3. The blood processing apparatus (1) of claim 2, wherein the flow tubes connected to at least two fluid chambers are connected to the dialysis fluid port (15).

4. The blood processing apparatus (1) of claim 2, wherein at least one flow tube connected to at least one of the n chambers is connected to either the first blood port (13) or the second blood port (14).

5. The blood processing apparatus (1) of claim 4, wherein the flow control unit (60) comprises:
a flow-blocking member (61) compressing a portion of the flow tube through which the flow control unit (60) controls a flow;
a flow-blocking wall (62) supporting the flow tube compressed by the flow-blocking member (61); and
a flow-blocking member driver providing a liner or curved movement force to the flow-blocking member (61).

6. The blood processing apparatus (1) of claim 4, wherein the flow control unit (60) comprises:
a flow control housing (64) having a cylinder-shaped internal space;
a flow control rotor (66) having a cylinder shape and disposed inside the internal space of the flow control housing (64);
a plurality of flow control ports (65) each penetrating the flow control housing (64) between the internal space and an outer surface thereof; and
a rotor driver driving the flow control rotor (66), wherein
a flow passage through at least one of the flow control ports (65) is blocked at any time, and an end of the flow control ports facing the flow control rotor (66) is placed within a cylinder surface of the flow control rotor (66).

7. The blood processing apparatus (1) of claim 4, wherein the flow control unit (60) comprises a one-way valve installed in at least one flow tube through which the flow control unit (60) controls a flow, thereby allowing a fluid to flow in one direction.

8. The blood processing apparatus (1) of claim 5, wherein the flow control unit (60) further comprises a one-way valve installed in at least one flow tube through which the flow control unit controls a flow, thereby allowing a fluid to flow in one direction.

9. The blood processing apparatus (1) of claim 5, wherein when the chambers are compressed or expanded, the flow control unit (60) blocks a flow through a half of the flow tubes through which the flow control unit controls a flow.

10. The blood processing apparatus (1) of claim 9, wherein the fluid chambers (51, 52, 53, 54, 55, 56, 57, 58) are made of an inflexible material, and
the chamber pressurizing member (59) includes a portion that is flexible to compress or expand the internal spaces of the chambers (51, 52, 53, 54, 55, 56, 57, 58).

11. The blood processing apparatus (1) of claim 9, wherein the fluid chamber (51, 52, 53, 54, 55, 56, 57, 58) is made of a flexible material that contracts and expands, and
the chamber pressurizing member (59) includes a portion that is inflexible to compress or expand the internal spaces of the chambers (51, 52, 53, 54, 55, 56, 57, 58).

12. The blood processing apparatus (1) of claim 9, wherein the fluid chamber (51, 52, 53, 54, 55, 56, 57, 58) is made of a flexible material that contracts and expands, and
the chamber pressurizing member (59) includes a pneumatic channel connected to the fluid chambers (51, 52, 53, 54, 55, 56, 57, 58) and through which the chambers (51, 52, 53, 54, 55, 56, 57, 58) are compressed or expanded due to an operation of a pneumatic driver pressurizing or depressurize the pneumatic channel.

13. The blood processing apparatus (1) of claim 4, further comprising a second flow control unit (60) provided in a flow tube connected to any one of the first blood port (13) or the second (15) blood port to open or block a flow therethrough.

14. The blood processing apparatus (1) of claim 1, wherein n is greater than 2.

## Patentansprüche

1. Blutverarbeitungsgerät (1), umfassend:
mehrere Flüssigkeitskammern, die jeweils einen Innenraum aufweisen;
ein Kammerdruckbeaufschlagungselement (59), das die Innenräume der Flüssigkeitskammern (51, 52, 53, 54, 55, 56, 57, 58) verdichtet oder ausdehnt;
einen Kammerdruckbeaufschlagungselementantrieb, der das Kammerdruckbeaufschlagungselement (59) antreibt; und
eine Durchflussregeleinheit (60), wobei
die mehreren Flüssigkeitskammern (51, 52, 53, 54, 55, 56, 57, 58) n Flüssigkeitskammern beinhalten (wobei n 2 oder mehr und eine positive ganze Zahl ist),
die n Flüssigkeitskammern jeweils mit einem ersten Durchflussschlauch, durch den eine Flüssigkeit an die Kammer bereitgestellt wird, und einem zweiten Durchflussschlauch, durch den eine Flüssigkeit der Kammer daraus abgeführt wird, verbunden sind und
die Durchflussregeleinheit (60) einen Durchfluss durch den ersten und den zweiten Durchflussschlauch, die mit den n Kammern verbunden sind, regelt, und
**dadurch gekennzeichnet, dass**, wenn das n eine gerade Zahl ist, gleichzeitig n/2 Kammern verdichtet und weitere n/2 Kammern ausgedehnt werden,
wobei, wenn das n eine ungerade Zahl ist, gleichzeitig (n+1)/2 Kammern verdichtet und (n-1)/2 Kammern ausgedehnt werden.

2. Blutverarbeitungsgerät (1) nach Anspruch 1, weiterhin umfassend einen Blutverarbeitungsfilter (10), in dem Blut verarbeitet wird, wobei der Blutverarbeitungsfilter (10) umfasst:
ein Filtergehäuse (11), das einen Innenraum aufweist;
einen ersten Blutport (13), der in einem Ende des Filtergehäuses (11) vorgesehen ist und durch den Blut an den Blutverarbeitungsfilter (10) bereitgestellt wird;
einen zweiten Blutport (14), der in einem anderen Ende des Filtergehäuses (11) vorgesehen ist und durch den Blut von dem Blutverarbeitungsfilter (10) abgeführt wird; und
mindestens einen Dialyseflüssigkeitsport (15), der an dem Filtergehäuse (11) angeordnet ist und durch den Dialyseflüssigkeit an den Blutverarbeitungsfilter (10) bereitgestellt und aus diesem abgeführt wird.

3. Blutverarbeitungsgerät (1) nach Anspruch 2, wobei die Durchflussschläuche, die mit mindestens zwei Flüssigkeitskammern verbunden sind, mit dem Dialyseflüssigkeitsport (15) verbunden sind.

4. Blutverarbeitungsgerät (1) nach Anspruch 2, wobei mindestens ein Durchflussschlauch, der mit mindestens einer der n Kammern verbunden ist, mit entweder dem ersten Blutport (13) oder dem zweiten Blutport (14) verbunden ist.

5. Blutverarbeitungsgerät (1) nach Anspruch 4, wobei die Durchflussregeleinheit (60) umfasst:
ein Durchflussblockadeelement (61), das einen Abschnitt des Durchflussschlauchs, durch den die Durchflussregeleinheit (60) einen Durchfluss regelt, zusammendrückt;
eine Durchflussblockadewand (62), die den Durchflussschlauch stützt, der durch das Durchflussblockadeelement (61) zusammengedrückt wird; und
einen Durchflussblockadeelementantrieb, der eine lineare oder gekrümmte Bewegungskraft an das Durchflussblockadeelement (61) bereitstellt.

6. Blutverarbeitungsgerät (1) nach Anspruch 4, wobei die Durchflussregeleinheit (60) umfasst:
ein Durchflussregelgehäuse (64), das einen zylinderförmigen Innenraum aufweist;
ein Durchflussregelrotor (66), die eine Zylinderform aufweist und im Inneren des Innenraums des Durchflussregelgehäuse (64) angeordnet ist;
mehrere Durchflussregelports (65), die jeweils das Durchflussregelgehäuse (64) zwischen dem Innenraum und einer Außenfläche davon durchdringen;
einen Rotorantrieb, der den Durchflussregelrotor (66) antreibt, wobei
eine Durchflusspassage durch mindestens einen der Durchflussregelports (65) jederzeit blockiert ist und ein Ende der Durchflussregelports, das dem Durchflussregelrotor (66) zugewandt ist, innerhalb einer Zylinderfläche des Durchflussregelrotors (66) platziert ist.

7. Blutverarbeitungsgerät (1) nach Anspruch 4, wobei die Durchflussregeleinheit (60) ein Einwegventil umfasst, das in mindestens einem Durchflussschlauch installiert ist, durch den die Durchflussregeleinheit (60) einen Durchfluss regelt, wodurch zugelassen wird, dass eine Flüssigkeit in einer Richtung fließt.

8. Blutverarbeitungsgerät (1) nach Anspruch 5, wobei die Durchflussregeleinheit (60) weiterhin ein Einwegventil umfasst, das in mindestens einem Durchflussschlauch installiert ist, durch den die Durchflussregeleinheit einen Durchfluss regelt, wodurch zugelassen wird, dass eine Flüssigkeit in einer Richtung fließt.

9. Blutverarbeitungsgerät (1) nach Anspruch 5, wobei, wenn die Kammern verdichtet oder ausgedehnt werden, die Durchflussregeleinheit (60) einen Durchfluss durch die Hälfte der Durchflussschläuche, durch die die Durchflussregeleinheit einen Durchfluss regelt, blockiert.

10. Blutverarbeitungsgerät (1) nach Anspruch 9, wobei die Flüssigkeitskammern (51, 52, 53, 54, 55, 56, 57, 58) aus einem unflexiblen Material hergestellt sind und
das Kammerdruckbeaufschlagungselement (59) einen Abschnitt beinhaltet, der flexibel ist, um die Innenräume der Kammern (51, 52, 53, 54, 55, 56, 57, 58) zu verdichten oder auszudehnen.

11. Blutverarbeitungsgerät (1) nach Anspruch 9, wobei die Flüssigkeitskammer (51, 52, 53, 54, 55, 56, 57, 58) aus einem flexiblen Material hergestellt ist, das sich zusammenzieht und ausdehnt, und
das Kammerdruckbeaufschlagungselement (59) einen Abschnitt beinhaltet, der unflexibel ist, um die Innenräume der Kammern (51, 52, 53, 54, 55, 56, 57, 58) zu verdichten oder auszudehnen.

12. Blutverarbeitungsgerät (1) nach Anspruch 9, wobei die Flüssigkeitskammer (51, 52, 53, 54, 55, 56, 57, 58) aus einem flexiblen Material hergestellt ist, das sich zusammenzieht und ausdehnt, und
das Kammerdruckbeaufschlagungselement (59) einen Pneumatikkanal beinhaltet, der mit den Flüssigkeitskammern (51, 52, 53, 54, 55, 56, 57, 58) verbunden ist und durch den die Kammern (51, 52, 53, 54, 55, 56, 57, 58) aufgrund eines Betriebs eines Pneumatikantriebs, der den Pneumatikkanal mit Druck beaufschlagt oder drucklos macht, verdichtet oder ausgedehnt werden.

13. Blutverarbeitungsgerät (1) nach Anspruch 4, weiterhin umfassend eine zweite Durchflussregeleinheit (60), die in einem Durchflussschlauch vorgesehen ist, der mit einem beliebigen von dem ersten Blutport (13) oder dem zweiten (15) Blutport verbunden ist, um einen Durchfluss dort hindurch zu öffnen oder zu blockieren.

14. Blutverarbeitungsgerät (1) nach Anspruch 1, wobei n größer als 2 ist.

## Revendications

1. Appareil de traitement sanguin (1) comprenant :
une pluralité de chambres de fluide ayant chacune un espace interne ;
un élément de pressurisation de chambre (59) comprimant ou dilatant les espaces internes des chambres de fluide (51, 52, 53, 54, 55, 56, 57, 58) ;
un dispositif d'entraînement d'élément de pressurisation de chambre entraînant l'élément de pressurisation de chambre (59) ; et
un module de commande d'écoulement (60), dans lequel
la pluralité de chambres de fluide (51, 52, 53, 54, 55, 56, 57, 58) inclut n chambres de fluide (où n est 2 ou plus et un entier positif),
les n chambres de fluide sont chacune connectées à un premier tube d'écoulement à travers lequel un fluide est fourni à la chambre et un second tube d'écoulement à travers lequel un fluide de la chambre est évacué de celle-ci, et
le module de commande d'écoulement (60) commande un écoulement à travers les premier et second tubes d'écoulement connectés aux n chambres, et
**caractérisé en ce que** lorsque le n est un nombre pair, n/2 chambres sont comprimées et n/2 autres chambres sont dilatées simultanément,
dans lequel lorsque le n est un nombre impair, (n+1)/2 chambres sont comprimées et (n-1)/2 chambres sont dilatées simultanément.

2. Appareil de traitement sanguin (1) selon la revendication 1, comprenant en outre un filtre de traitement sanguin (10) dans lequel du sang est traité, dans lequel le filtre de traitement sanguin (10) comprend :
un logement de filtre (11) ayant un espace interne ;
un premier port sanguin (13) prévu dans une extrémité du logement de filtre (11) et à travers lequel du sang est fourni au filtre de traitement sanguin (10) ;
un second port sanguin (14) prévu dans une autre extrémité du logement de filtre (11) et à travers lequel du sang est évacué du filtre de traitement sanguin (10) ; et
au moins un port de fluide de dialyse (15) disposé au niveau du logement de filtre (11) et à travers lequel du fluide de dialyse est fourni au ou évacué du filtre de traitement sanguin (10).

3. Appareil de traitement sanguin (1) selon la revendication 2, dans lequel les tubes d'écoulement connectés à au moins deux chambres de fluide sont connectés au port de fluide de dialyse (15).

4. Appareil de traitement sanguin (1) selon la revendication 2, dans lequel au moins un tube d'écoulement connecté à au moins une des n chambres est connecté au premier port sanguin (13) ou au second port sanguin (14).

5. Appareil de traitement sanguin (1) selon la revendication 4, dans lequel le module de commande d'écoulement (60) comprend :
un élément de blocage d'écoulement (61) comprimant une portion du tube d'écoulement à travers lequel le module de commande d'écoulement (60) commande un écoulement ;
une paroi de blocage d'écoulement (62) supportant le tube d'écoulement comprimé par l'élément de blocage d'écoulement (61) ; et
un dispositif d'entraînement d'élément de blocage d'écoulement fournissant une force à mouvement linéaire ou incurvé à l'élément de blocage d'écoulement (61).

6. Appareil de traitement sanguin (1) selon la revendication 4, dans lequel le module de commande d'écoulement (60) comprend :
un logement de commande d'écoulement (64) ayant un espace interne en forme de cylindre ;
un rotor de commande d'écoulement (66) ayant une forme de cylindre et disposé à l'intérieur de l'espace interne du logement de commande d'écoulement (64) ;
une pluralité de ports de commande d'écoulement (65) pénétrant chacun dans le logement de commande d'écoulement (64) entre l'espace interne et une surface externe de celui-ci ; et
un dispositif d'entraînement de rotor entraînant le rotor de commande d'écoulement (66), dans lequel
un passage d'écoulement à travers au moins un des ports de commande d'écoulement (65) est bloqué à tout moment, et une extrémité des ports de commande d'écoulement tournée vers le rotor de commande d'écoulement (66) est placée au sein d'une surface cylindrique du rotor de commande d'écoulement (66).

7. Appareil de traitement sanguin (1) selon la revendication 4, dans lequel le module de commande d'écoulement (60) comprend une valve antiretour installée dans au moins un tube d'écoulement à travers laquelle le module de commande d'écoulement (60) commande un écoulement, permettant de ce fait à un fluide de s'écouler dans une direction.

8. Appareil de traitement sanguin (1) selon la revendication 5, dans lequel le module de commande d'écoulement (60) comprend en outre une valve antiretour installée dans au moins un tube d'écoulement à travers laquelle le module de commande d'écoulement commande un écoulement, permettant de ce fait à un fluide de s'écouler dans une direction.

9. Appareil de traitement sanguin (1) selon la revendication 5, dans lequel lorsque les chambres sont comprimées ou dilatées, le module de commande d'écoulement (60) bloque un écoulement à travers une moitié des tubes d'écoulement à travers laquelle le module de commande d'écoulement commande un écoulement.

10. Appareil de traitement sanguin (1) selon la revendication 9, dans lequel les chambres de fluide (51, 52, 53, 54, 55, 56, 57, 58) sont réalisées en un matériau inflexible, et
l'élément de pressurisation de chambre (59) inclut une portion qui est flexible pour comprimer ou dilater les espaces internes des chambres (51, 52, 53, 54, 55, 56, 57, 58).

11. Appareil de traitement sanguin (1) selon la revendication 9, dans lequel la chambre de fluide (51, 52, 53, 54, 55, 56, 57, 58) est réalisée en un matériau flexible qui se contracte et se dilate, et
l'élément de pressurisation de chambre (59) inclut une portion qui est inflexible pour comprimer ou dilater les espaces internes des chambres (51, 52, 53, 54, 55, 56, 57, 58).

12. Appareil de traitement sanguin (1) selon la revendication 9, dans lequel la chambre de fluide (51, 52, 53, 54, 55, 56, 57, 58) est réalisée en un matériau flexible qui se contracte et se dilate, et
l'élément de pressurisation de chambre (59) inclut un canal pneumatique connecté aux chambres de fluide (51, 52, 53, 54, 55, 56, 57, 58) et à travers lequel les chambres (51, 52, 53, 54, 55, 56, 57, 58) sont comprimées ou dilatées en raison d'une opération d'un dispositif d'entraînement pneumatique pressurisant ou dépressurisant le canal pneumatique.

13. Appareil de traitement sanguin (1) selon la revendication 4, comprenant en outre un second module de commande d'écoulement (60) prévu dans un tube d'écoulement connecté à l'un quelconque du premier port sanguin (13) ou du second port sanguin (15) pour ouvrir ou bloquer un écoulement à travers lui.

14. Appareil de traitement sanguin (1) selon la revendication 1, dans lequel n est supérieur à 2.
